(19) Europäisches Patentamt
European Patent Office
Office européen des brevets

(11) **EP 0 927 214 B2**

(12) **NEW EUROPEAN PATENT SPECIFICATION**
After opposition procedure

(45) Date of publication and mention
of the opposition decision:
**12.03.2014   Bulletin 2014/11**

(45) Mention of the grant of the patent:
**11.07.2007   Bulletin 2007/28**

(21) Application number: **97944351.2**

(22) Date of filing: **23.09.1997**

(51) Int Cl.:
**C08G 65/00** (2006.01)       **A61K 9/00** (2006.01)
**C08G 85/00** (2006.01)

(86) International application number:
**PCT/US1997/016857**

(87) International publication number:
**WO 1998/012243 (26.03.1998 Gazette 1998/12)**

(54) **POLYMERIZABLE BIODEGRADABLE POLYMERS INCLUDING CARBONATE OR DIOXANONE LINKAGES**

POLYMERISIERBARE BIOLOGISCH ABBAUBARE POLYMERE MIT CARBONAT-ODER DIOXANONBINDUNGEN

POLYMERES BIODEGRADABLES POLYMERISABLES CONTENANT DES LIAISONS CARBONATES OU DIOXANONES

(84) Designated Contracting States:
**AT BE CH DE DK ES FI FR GB GR IE IT LI LU MC NL PT SE**

(30) Priority: **23.09.1996   US 710689**
**06.08.1997   US 54849 P**

(43) Date of publication of application:
**07.07.1999   Bulletin 1999/27**

(73) Proprietor: **GENZYME CORPORATION**
**Cambridge, MA 02142 (US)**

(72) Inventors:
• **JARRETT, Peter, K.**
**Sudbury, MA 01776 (US)**
• **SAWHNEY, Amarpreet**
**Bedford, MA 01730 (US)**
• **COURY, Arthur, J.**
**Boston, MA 02116 (US)**
• **RUDOWSKY, Ronald, S.**
**Sudbury, MA 01776 (US)**
• **POWELL, Michelle, D.**
**Tewksbury, MA 01876 (US)**
• **AVILA, Luis, Z.**
**Arlington, MA 02174 (US)**
• **ENSCORE, David, J.**
**Sudbury, MA 01776 (US)**
• **GOODRICH, Stephen, D.**
**Woburn, MA 01801 (US)**
• **NASON, William, C.**
**Westford, MA 01886 (US)**
• **YAO, Fei**
**North Andover, MA 01845 (US)**
• **WEAVER, Douglas**
**Bedford, MA 01730 (US)**
• **BARMAN, Shikha, P.**
**Bedford, MA 01730 (US)**

(74) Representative: **Potter Clarkson LLP**
**The Belgrave Centre**
**Talbot Street**
**Nottingham, NG1 5GG (GB)**

(56) References cited:
**EP-A- 0 258 749       WO-A-96/11671**
**WO-A1-98/00170       US-A- 5 410 016**

• **W.N.E. VAN DIJK-WOLTHUIS ET AL.:
'Degradation Kinetics of Methacrylated Dextrans in Aqueous Solution' JOURNAL OF PHARMACEUTICAL SCIENCES vol. 86, no. 4, 04 April 1997, pages 413 - 417**
• **W.E. HENNINK ET AL.: 'Controlled release of proteins from dextran hydrogels' JOURNAL OF CONTROLLED RELEASE vol. 39, 1996, GENT, BELGIUM, pages 47 - 55**
• **J.HELLER ET AL.: 'Controlled release of water-soluble macromolecules from bioerodible hydrogels' BIOMATERIALS vol. 4, October 1983, pages 262 - 266**

Remarks:
The file contains technical information submitted after the application was filed and not included in this specification

## Description

### Field of the invention

The present invention relates to improved photopolymerizable biodegradable hydrogels for use as tissue adhesives, coatings, sealants and in controlled drug delivery devices. The improved materials incorporate trimethylene carbonate and/or dioxanone linkages. These biodegradable linkages allow improved control of various properties of the macromers, particularly increasing viscosity while preserving biodegradability.

### Background of the Invention

U.S. Patent No. 5,410,016 to Hubbell et al. discloses biocompatible, biodegradable macromers which can be polymerized to form hydrogels. The macromers are block copolymers that include a biodegradable block, a water-soluble block with sufficient hydrophilic character to make the macromer water-soluble, and one or more polymerizable groups. The polymerizable groups are separated from each other by at least one degradable group, Hubbell specifically discloses using polyhydroxy acids, such as polylactide, polyglycolide and polycaprolactone as the biodegradable polymeric blocks. One of the disclosed uses for the macromers is to plug or seal leaks in tissue.

Other hydrogels have been described, for example, in U.S. Patent No. 4,938,763 to Dunn et al., U.S. Patent Nos. 5,100,992 and 4,826,945 to Cohn et al., U.S. Patent Nos. 4,741,872 and 5,160,745 to De Luca et al., U.S. Patent No. 5,527,864 to Suggs et al., and U.S. Patent No. 4,511,478 to Nowinski et al. Methods of using such polymers are described in U.S. Patent No. 5,573,934 to Hubbell et al. and PCT WO 96/29370 by Focal.

While numerous references disclose using homopolymers and copolymers including carbonate linkages to form solid medical devices, such as sutures, suture coatings and drug delivery devices (see, for example, U.S. Patent No. 3,301,824 to Hostettler et al., U.S. Patent No. 4,243,775 to Rosensaft et al., U.S. Patent No. 4,429,080 to Casey et al., U.S. Patent No. 4,716,20 to Casey et al., U.S. Patent No. 4,857,602 to Casey et al., U.S. Patent No. 4,882,168 to Casey, EP 0 390 860 B1 by Boyle et al., U.S. Patent No. 5,066,772 to Tang et al., U.S. Patent No. 5,366,756 to Chesterfield et al., U.S. Patent No. 5,403,347 to Roby et al. and U.S. Patent No. 5,522,841 to Roby et al.), none of these publications discloses incorporating polymerizable groups on the polymers so that the polymers can be further polymerized. Accordingly, none of these polymers can be used in the same manner as the macromers in U.S. Patent No. 5,410,016 to Hubbell et al.

Sealing or plugging holes in lung tissue is inherently more difficult than sealing other types of tissue because the tissue is constantly expanded and contracted during normal respiration. It would be advantageous to provide macromers which can be rapidly polymerized *in vivo* to form hydrogels which are more elastic than conventional hydrogels, for example, for use in sealing lung tissue.

It is therefore an object of the present invention to provide biodegradable, biocompatible macromers that can be rapidly polymerized *in vivo* to form hydrogels which are more elastic than conventional hydrogels.

It is a further object of the present invention to provide a macromer solution which can be administered during surgery or outpatient procedures and polymerized as a tissue adhesive, cell encapsulating medium, tissue sealant, wound dressing or drug delivery device.

It is a still further object of the present invention to provide a macromer solution which can be polymerized *in vivo* on a surface to be coated in a very short time frame to form conformal coating layers.

### Summary of the Invention

Biocompatible, biodegradable, polymerizable and at least substantially water-soluble macromers and methods of preparation and use thereof are disclosed. The macromers are block copolymers that include at least one water-soluble block, at least one biodegradable block, and at least one polymerizable group. At least one of the biodegradable blocks comprises a linkage based on a trimethylene carbonate or dioxanone group, and the macromers can contain other degradable linkages or groups in addition to trimethylene carbonate or dioxanone.

The carbonate and dioxanone linkages impart more elasticity to the polymer and degrade at a different rate than hydroxy acid linkages. Carbonate linkages can also increase macromer viscosity, at a given concentration, without requiring increased molecular weight of the nondegradable components of the macromer. The macromers can also include poly(hydroxy acid) linkages which degrade by hydrolysis into relatively non-toxic hydroxy acid residues, or other biodegradable blocks such as polycaprolactones, polyorthoesters, polyanhydrides, and polypeptides. The degradation time of the polymers can be controlled, for example, by selecting the types and proportion of the biodegradable blocks.

The polymerizable groups can be polymerized by either free radical (homolytic) processes or by heterolytic processes (such as cationic polymerization). Preferably, the groups are polymerized photochemically. The macromer can be polymerized in the presence of prophylactic, therapeutic or diagnostic agents, for delivery of the incorporated agents in a controlled manner as the resulting polymer degrades. The macromers are useful for delivering hydrophobic, hydrophilic and/or labile materials. They can be particularly useful for delivery of hydrophobic materials.

The macromers can be polymerized in an interfacial manner to form ultrathin coatings which are intimately adhered to the coated surface, or in a bulk manner to form relatively thick coatings which may or may not be intimately adhered to the coated surface. Alternatively, the two methods can be combined to provide a relatively thick coating which is intimately adhered to the surface. Each of these methods is advantageous for certain applications. The present invention relates to a macromer as defined in claim 1 to 24. In one aspect, the present invention relates to a use as defined in claim 25 and a second medical use as defined in claim 26. A method for making a device is defined in claim 27 and a further embodiment of this method is defined in claim 28. A further use is defined in claim 29. A method for making a macromer is defined in claim 30. The use of the macromer to manufacture a compliant polymeric material is defined in claim 31. A biodegradable polymer is defined in claim 32. A use of a compliant material is defined in claim 33. A use of the polymer is defined in claim 34.

**Brief Description of the Figures**

Figure 1 is a graph of the elastic strength (seal pressure, mm Hg) over time (hr) of five different sealant materials: 10% 35K T, 20% 35K T, 10% 20K TL, 10% 20 K TL, and 20% 35K TL K is defined as 100 Daltons (weight average molecular weight, T is trimethylene carbonate (TMC), L is lactide, and TL is a copolymer of TMC and lactide.

Figures 2A and 2B are graphs of the degradation (% mass loss) over time (days) for 20K T (Figure 2A) and 35K T (Figure 2B) for subcutaneous polymeric implants in rats.

Figure 3 shows the stress vs. strain curve of a compliant sealant formed by photopolymerization of a poly(ethylene glycol)-oligotrimethylene carbonate copolymer end capped with acrylate ester.

**Detailed Description of the Invention**

Definitions

As used herein, the term "sealant" refers to a material which decreases or prevents the migration of fluid from or into a surface such as a tissue surface. Sealants are typically formed by the application of precursor molecules to a tissue followed by local polymerization. The same materials may also be used to adhere materials together, either when applied between them and polymerized, or when used to jointly embed materials.

As used herein, the term "biocompatibility," in the context of biologically-related uses, refers to the absence of stimulation of a severe, long-lived or escalating biological response to an implant or coating, and is distinguished from a mild, transient inflammation which typically accompanies surgery or implantation of foreign objects into a living organism.

As used herein the term "biodegradability" refers to the disintegration, which is preferably predictable, of an implant into small entities which will be metabolized or excreted, under the conditions normally present in a living tissue.

The properties of the particular coating or barrier materials disclosed herein are referred to as "materials properties", and include:

the "Young's modulus" (of elasticity) which is the limiting modulus of elasticity extrapolated to zero strain; the "elastic modulus" which is any modulus of elasticity, not limited to Young's modulus, and may include "secant modulus" and other descriptors of non-linear regions of the stress-strain curve; the "bulk" or "compressive" modulus which is used in its usual sense of ratio of stress to a designated compressive strain; the "elongation at failure" which is the relative strain or extension of a test specimen at which any irreversible or hysteresis-inducing change occurs in the specimen; and the "elongation at break" or "elongation at rupture" which is the relative strain (extension) of a test specimen at which mechanical rupture occurs.

The term "compliance" as used herein is used in a general sense, and refers for example to the ability of an implant to closely match the physiological and mechanical properties of tissues at the implant site, except when "compliance" is used in a specific technical sense as the reciprocal of a modulus.

As applied to a relatively thin, flat material such as a tissue or a layer of sealant, "normalized compliance" (NC) is defined herein as the strain,(i.e., the elongation or compression per unit length of a specimen), divided by the applied force per unit cross-sectional area, further divided by the thickness of the specimen. Hence, for a sample having a width w (for example, the width of the clamps of the testing apparatus), and a thickness t, when an applied force F produces a strain S, then the compliance C is

$$C = \frac{S}{F/wt} = \frac{S \cdot wt}{F}$$

and the normalized compliance is

$$NC = \frac{C}{t} = \frac{S}{F/w} = \frac{Sw}{F}$$

*i.e.*, the strain in the sample divided by the force per unit width applied to the sample. The normalized compliance allows direct comparison of the forces required to deform the tissue versus a coating on the tissue, without regard to the relative thicknesses of these materials.

The normalized compliance ratio (abbreviated NCR) is defined as the value of the normalized compliance of the tissue or other substrate divided by the normalized compliance of the sealant material. When both measurements are conducted on strips of the same width and at the same force, the NCR is simply the ratio of the strains at a particular force. A low NCR (less than 1) is obtained when the sealant material is easier to deform than the tissue, while a high NCR (greater than 1) is obtained when the tissue is easier to deform than the sealing material.

As used herein, the term "elastomer" refers to a polymeric material which at room temperature is capable of repeatedly recovering in size and shape after removal of a deforming force. In some embodiments, an elastomer is a material which can be repeatedly stretched to twice its original length and will repeatedly return to its approximate length on release of the stress.

The phrase "elastomeric materials" is a phrase which has been used in the literature. There are many publications describing structure-property relationships of elastomers and other deformable materials. Lower elastic modulus and, frequently, an increased reversible elongation to break or fracture, are found when any of the following occur:

1. The distance between nodes or junctions or more crystalline ("hard") segments increases.
2. The crosslink density decreases. This may be controlled by amount of crosslinker, nature of crosslinker, and degree of cure, as well as by segment length of either the crosslinked species or the crosslinking species, where different.
3. For a material at equilibrium with a continuous phase, an increase in the plasticization of the elastomer by the continuous phase. For applications wherein the continuous phase is water, more particularly physiological saline, increasing hydrophilicity tends to increase compliance.

In order to seal fluid leaks in tissue, the sealing material must remain firmly bonded to the tissue during motions required of the tissue during the healing process. For tissues and organs which cannot be immobilized, such as the lung, an effective sealing material is both tightly-adherent and compliant, having materials properties similar to those of the tissue. Examples of compliant adherent materials and methods for their construction and use are provided.

In one embodiment, one or more initiators are applied to a surface to form an absorbed layer. "Absorbed" is used herein to encompass both "absorbed" and "adsorbed". A solution of polymerizable molecules, referred to herein as "monomers", is then applied.

## **Methods**

In one embodiment, one or more initiators or components of an initiation system are applied directly to the surface, and the unabsorbed excess is optionally removed by washing or blotting. The initiator solution may further contain one or more polymerizable monomers, and other useful formulating ingredients, including accelerators, co-initiators, sensitizers, and co-monomers. Then a liquid containing polymerizable monomers in combination with one or more initiators or components of an initiation system, which may be the same as or different from that absorbed in the first step, is applied. The system, if not self-polymerizing, is then stimulated to polymerize, for example by application of an appropriate wavelength of light.

The priming and monomer-application steps can also be combined. For example, if excess initiator is not removed before monomer addition, then subsequent application of monomer will result in mixture of initiator into the monomer layer. Similarly, if the monomer layer contains an initiator with a high affinity for the surface, then it is possible to apply a monomer layer containing initiator, and wait an appropriate time to allow preferential absorption of the initiator to the surface, to achieve the same effect.

All of these methods may collectively be described as application of the monomer in an "initiating-incorporating manner", encompassing any means of application and mixing which results in both an absorbed layer of initiator, and a layer of monomer incorporating an initiator, being present on a surface to be coated.

The initiators may be chemical, photochemical, or a combination thereof. With non-photochemical systems, a reductant component and an oxidant component may be present in the two parts of the solution, i.e., in the priming layer and the coating layer.

Alternatively, a two-step process can be used to form polymers, especially bioabsorbable hydrogels on tissue. In the first step the tissue is treated with an initiator or a part of an initiator system for the polymerization of olefinic (e.g. acrylic) or other functional monomers, optionally with monomer in the priming solution. This provides an activated tissue surface. In the second step, monomer(s) and, if appropriate, the remainder of an initiator system, are together placed in contact with the activated tissue, resulting in polymerization on the tissue. An example of such a system is the combination of a peroxygen compound in one part, and a reactive ion, such as a transition metal, in another.

This process of spontaneous polymerization does not require the use of a separate energy source. Moreover, since the process of polymerization is initiated when part one contacts part two, there are no "pot life" issues due to initiation of polymerization. If desired, part one or part two can contain dyes or other means for visualizing the hydrogel coating.

An example of a system that can be used in this method is the spontaneous "contact" initiator systems such as those found in two part "acrylic structural adhesives". All components of the materials used as described herein, however, must display biocompatibility as well as the ability to spontaneously polymerize on tissue. The use of tributyl borane for this purpose is illustrated here.

These systems can markedly simplify the delivery of gel to tissue, especially in areas hard to reach or hold for a photochemical system. The delivery system can be much simpler. Moreover, it has been discovered that a two-part chemical system such as a redox system and especially one based on peroxygen, can be used to chemically enhance the curing of a photochemical system, thereby combining the control of a photochemical system with the ability of a chemical system to overcome colored impurities, such as blood.

## Polymers

Water-soluble, biocompatible, biodegradable macromers and methods of preparation and use thereof, are disclosed. The macromers include at least one water-soluble block, at least one biodegradable block, and at least one polymerizable group. At least one biodegradable block contains a trimethylene carbonate or dioxanone group. To obtain a biodegradable material after polymerization, each polymerizable group must be separated from any other polymerizable group on the macromer by at least one biodegradable linkage or group.

At least a portion of the macromers will contain more than one reactive group and thereby be effective as crosslinkers, so that the macromers can be crosslinked to form a gel. The minimal proportion required will vary with the nature of the macromer and its concentration in solution, and the proportion of crosslinker in the macromer solution can be as high as 100% of the macromer solution.

For example, the macromers include at least 1.02 polymerizable groups on average, and, more preferably, the macromers each include two or more polymerizable groups on average.

Since in the preferred homolytic (free radical) polymerization reactions each polymerizable group will polymerize into a chain, crosslinked hydrogels can be produced using only slightly more than one reactive group per macromer (i.e., about 1.02 polymerizable groups on average). However, higher percentages are preferable, and excellent gels can be obtained in polymer mixtures in which most or all of the molecules have two or more reactive double bonds. Poloxamines, an example of a water-soluble block, have four arms and thus may readily be modified to include four polymerizable groups.

As used herein, a "biocompatible" material is one which stimulates only a mild, often transient, implantation response, as opposed to a severe or escalating response.

As used herein, a "biodegradable" material is one which decomposes under normal *in vivo* physiological conditions into components which can be metabolized or excreted.

As used herein, a "block" is a region of a copolymer differing in subunit composition from neighboring regions. Blocks will generally contain multiple subunits, up to about one thousand subunits or less for non-degradable materials, and without an upper limit for degradable materials. In the lower limit, the size of a block depends on its function; the minimum size is that which is sufficient to allow the function to be performed. In the case of a block conferring water-solubility on the macromer, this will be typically 400 daltons or more, preferably 600 daltons or more, more preferably at least 1000 daltons, and most preferably in the range of 2000 to 40,000 daltons. For degradable linkages, the minimum block size is a single linkage of the appropriate degradability for the function. More preferably, the block size is two to forty groups; most preferably, three to twenty. The reactive groups may be considered as a block for some purposes;

the typical number of units in such a block is one, but may be two to five.

As used herein, a dioxanone is a repeating unit with the structure -O-C(O)-R-O-, where R is a straight, branched or cyclic alkyl group. An example of a cyclic dioxanone is 1,4-dioxan-2-one. 1,4-dioxan-2-one is a preferred dioxanone.

As used herein, a hydrogel is a substance formed when an organic polymer (natural or synthetic) is cross-linked via covalent, ionic, or hydrogen bonds to create a three-dimensional open-lattice structure which entraps water molecules to form a gel.

As used herein, "water-soluble" is defined as a solubility of at least one gram/liter in an aqueous solution at a temperature in the range of about 0° C and 50°C. Aqueous solutions can include small amounts of water-soluble organic solvents, such as dimethylsulfoxide, dimethylformamide, alcohols, acetone, and/or glymes.

**Types of Block Copolymers**

In general terms, the macromers are block co-polymers that comprise a biodegradable block, a water-soluble block, and at least one polymerizable group. Preferably, the macromers comprise at least 1.02 polymerizable groups on average, and, more preferably, include at least two polymerizable groups per macromer, on average. Average numbers of polymerizable groups can be obtained, for example, by blending macromers with different amounts of polymerizable groups.

The individual polymeric blocks can be arranged to form different types of block copolymers, including di-block, tri-block, and multi-block copolymers. The polymerizable blocks can be attached directly to biodegradable blocks or indirectly via water-soluble nondegradable blocks, and are preferably attached so that the polymerizable groups are separated from each other by a biodegradable block. For example, if the macromer contains a water-soluble block coupled to a biodegradable block, one polymerizable group may be attached to the water-soluble block and another attached to the biodegradable block. Preferably, both polymerizable groups would be linked to the water-soluble block by at least one degradable linkage.

The di-block copolymers include a water-soluble block linked to a biodegradable block, with one or both ends capped with a polymerizable group. The tri-block copolymers can include a central water-soluble block and outside biodegradable blocks, with one or both ends capped with a polymerizable group. Alternatively, the central block can be a biodegradable block, and the outer blocks can be water-soluble. The multiblock copolymers can include one or more of the water-soluble blocks and biocompatible blocks coupled together in a linear fashion. Alternatively, the multiblock copolymers can be brush, comb, dendritic or star copolymers. If the backbone is formed of a water-soluble block, at least one of the branches or grafts attached to the backbone is a biodegradable block. Alternatively, if the backbone is formed of a biodegradable block, at least one of the branches or grafts attached to the backbone is a water-soluble block, unless the biodegradable block is also water-soluble. In another embodiment, a multifunctional compound, such as a polyol, can be coupled to multiple polymeric blocks, at least one of which is water-soluble and at least one of which is biodegradable.

In general, any formulation of the macromer which is intended to be biodegradable must be constructed so that each polymerizable group is separated from each other polymerizable group by one or more linkages which are biodegradable. Non-biodegradable materials are not subject to this constraint.

Those skilled in the art will recognize that the individual polymeric blocks may have uniform compositions, or may have a range of molecular weights, and may be combinations of relatively short chains or individual species which confer specifically desired properties on the final hydrogel, while retaining the required characteristics of the macromer. The lengths of oligomers referred to herein may vary from single units (in the biodegradable portions) to many, subject to the constraint of preserving the overall water-solubility of the macromer.

In the discussion below and the examples, macromers are often designated by a code of the form xxKZn. xxK represents the molecular weight of the backbone polymer, which is polyethylene glycol ("PEG") unless otherwise stated, in thousands of Daltons. Z designates the biodegradable linkage, using a code wherein where L is for lactic acid, G is for glycolic acid, D is for dioxanone, C is for caprolactone, T is for trimethylene carbonate, and n is the average number of degradable groups in the block. The molecules are terminated with acrylic ester groups, unless otherwise stated. This is sometimes also indicated by the suffix A2.

While the preferred biodegradable groups (in addition to trimethylene carbonate or dioxanone) are hydroxy acids, orthoesters, anhydrides, or other synthetic or semisynthetic degradable linkages, natural materials may be used in the biodegradable sections when their degree of degradability is sufficient for the intended use of the macromer. Such biodegradable groups may comprise natural or unnatural amino acids, carbohydrate residues, and other natural linkages. Biodegradation time will be controlled by the local availability of enzymes hydrolyzing such linkages. The availability of such enzymes may be ascertained from the art or by routine experimentation.

**Water soluble regions.**

Suitable water-soluble polymeric blocks include those prepared from poly(ethylene glycol), poly(ethylene oxide), partially or fully hydrolyzed poly(vinyl alcohol), poly(vinylpyrrolidone), poly(ethyloxazoline), poly(ethylene oxide)-co-poly (propylene oxide) block copolymers (poloxamers and meroxapols), poloxamines, carboxymethyl cellulose, hydroxyalkylated celluloses such as hydroxyethyl cellulose and methylhydroxypropyl cellulose, polypeptides, polynucleotides, polysaccharides or carbohydrates such as Ficoll® polysucrose, hyaluronic acid, dextran, heparan sulfate, chondroitin sulfate, heparin, or alginate, and proteins such as gelatin, collagen, albumin, or ovalbumin. Preferably, the water-soluble polymeric blocks are made from poly(ethylene glycol) or poly(ethylene oxide).

The soluble polymer blocks may be intrinsically biodegradable or may be poorly biodegradable or effectively non-biodegradable in the body. In the latter two cases, the soluble blocks should be of sufficiently low molecular weight to allow excretion. The maximum molecular weight to allow excretion in human beings (or other species in which use is intended) will vary with polymer type, but will often be about 40,000 daltons or below. Water-soluble natural polymers and synthetic equivalents or derivatives, including polypeptides, polynucleotides, and degradable polysaccharides, can be used.

The water-soluble blocks can be a single block with a molecular weight of at least 600, preferably 2000 or more, and more preferably at least 3000 Daltons. Alternatively, the water-soluble blocks can be two or more water-soluble blocks which are joined by other groups. Such joining groups can include biodegradable linkages, polymerizable linkages, or both. For example, an unsaturated dicarboxylic acid, such as maleic, fumaric, or aconitic acid, can be esterified with degradable groups as described below, and such linking groups can be conjugated at one or both ends with hydrophilic groups such as polyethylene glycols.

**Biodegradable Blocks.**

The biodegradable blocks are preferably hydrolyzable under *in vivo* conditions. At least one biodegradable region is a trimethylene carbonate or dioxanone linkage. Additional biodegradable polymeric blocks can include polymers and oligomers of hydroxy acids or other biologically degradable polymers that yield materials that are non-toxic or present as normal metabolites in the body. Preferred poly(hydroxy acid)s are poly(glycolic acid), poly(DL-lactic acid) and poly (L-lactic acid). Other useful materials include poly(amino acids), poly(anhydrides), poly(orthoesters), and poly(phosphoesters). Polylactones such as poly(epsilon-caprolactone), poly(delta-valerolactone), poly(gamma-butyrolactone)and poly (beta-hydroxybutyrate), for example, are also useful.

Biodegradable regions can be constructed from monomers, oligomers or polymers using linkages susceptible to biodegradation, such as ester, peptide, anhydride, orthoester, and phosphoester bonds.

By varying the total amount of biodegradable groups, and selecting the ratio between the number of dioxanone linkages (which are relatively slow to hydrolyze) and of lower hydroxy acid linkages (especially glycolide or lactide, which hydrolyze relatively rapidly), the degradation time of hydrogels formed from the macromers can be controlled.

**Dioxanones**

Suitable dioxanones include dioxanone (p-dioxanone; 1,4-dioxan-2-one; 2-keto-1,4-dioxane), and the closely related materials 1,4-dioxolan-2-one, 1,4-dioxepan-2-one and 1,5-dioxepan-2-one. Lower alkyl, for example C1-C4 alkyl, derivatives of these compounds are also contemplated, such as 2-methyl p-dioxanone (cyclic O-hydroxyethyl ether of lactic acid).

**Polymerizable Groups.**

The term "polymerizable group" is defined as a reactive functional group that has the capacity to form additional covalent bonds resulting in macromer interlinking. Polymerizable groups specifically include groups capable of polymerizing via free radical polymerization and groups capable of polymerizing via cationic or heterolytic polymerization. Suitable groups include, but are not limited to, ethylenically or acetylenically unsaturated groups, isocyanates, epoxides (oxiranes), sulfhydryls, succinimides, maleimides, amines, imines, amides, carboxylic acids, sulfonic acids and phosphate groups. (Aliphatic hydroxy groups are not considered to be reactive groups for the chemistry disclosed herein, except in formulations which also contain groups capable of covalent crosslinking with such hydroxyls.) Ethylenically unsaturated groups include vinyl groups such as vinyl ethers, N-vinyl amides, allyl groups, unsaturated monocarboxylic acids, unsaturated dicarboxylic acids, and unsaturated tricarboxylic acids. Unsaturated monocarboxylic acids include acrylic acid, methacrylic acid and crotonic acid. Unsaturated dicarboxylic acids include maleic, fumaric, itaconic, mesaconic or citraconic acid. Unsaturated tricarboxylic acids include aconitic acid. Polymerizable groups may also be derivatives of such materials, such as acrylamide, N-isopropylacrylamide, luydroxyethylacrylate, hydroxyethylmethacrylate, and analogous vinyl and allyl compounds. Reactive group forming compounds will preferably be available in a stable activated form, to allow simple incorporation into the macromer. Examples of such materials are (meth)acrylyl chloride, acrylic anhydride, and allyl glycidyl ether. The polymerizable groups are preferably located at one or more ends of the macromer. In a less preferred embodiment, the polymerizable groups can be located within the macromer.

Polymerization is initiated by any convenient reaction, including photopolymerization, chemical or thermal free-radical polymerization, redox reactions, cationic polymerization, and chemical reaction of active groups (such as isocyanates, for example.) Polymerization is preferably initiated using photoinitiators. Photoinitiators that generate a free radical or a .cation on exposure to UV light are well known to those of skill in the art. Free-radicals can also be formed in a relatively mild manner from photon absorption of certain dyes and chemical compounds. The polymerizable groups are preferably polymerizable by free radical polymerization. The preferred polymerizable groups are acrylates, diacrylates, oligoacrylates, methacrylates, dimethacrylates, oligomethacrylates, cinnamates, dicinnamates, oligocinnamates, and other biologically acceptable photopolymerizable groups.

These groups can be polymerized using photoinitiators that generate free radicals upon exposure to light, including UV (ultraviolet) and IR (infrared) light, preferably long-wavelength ultraviolet light (LWUV) or visible light. LWUV and visible light are preferred because they cause less damage to tissue and other biological materials than short-wave UV light. Useful photoinitiators are those which can be used to initiate polymerization of the macromers without cytotoxicity and within a short time frame, minutes at most and most preferably seconds.

Exposure of dyes, preferably in combination with co-catalysts such as amine, to light, preferably visible or LWUV light, can generate free radicals. Light absorption by the dye causes the dye to assume a triplet state, and the triplet state subsequently reacts with the amine to form a free radical which initiates polymerization, either directly or via a suitable electron transfer reagent or co-catalyst, such as an amine. Polymerization can be initiated by irradiation with light at a wavelength of between about 200-1200 nm, most preferably in the long wavelength ultraviolet range or visible range, 320 nm or higher, and most preferably between about 365 and 550 nm.

Numerous dyes can be used for photopolymerization. Suitable dyes are well known to those of skill in the art. Preferred dyes include erythrosin, phloxime, rose bengal, thionine, camphorquinone, ethyl eosin, eosin, methylene blue, riboflavin, 2,2-dimethyl-2-phenylacetophenone, 2-methoxy-2-phenylacetophenone, 2,2-dimethoxy-2-phenyl acetophenone, other acetophenone derivatives, and camphorquinone. Suitable cocatalysts include amines such as N-methyl diethanolamine, N,N-dimethyl benzylamine, triethanol amine, triethylamine, dibenzyl amine, N-benzylethanolamine, N-isopropyl benzylamine. Triethanolamine is a preferred cocatalyst.

Suitable chemical, thermal and redox systems may initiate the polymerization of unsaturated groups by generation of free radicals in the initiator molecules, followed by transfer of these free radicals to the unsaturated groups to initiate a chain reaction. Peroxides and other peroxygen compounds are well-known in this regard, and may be considered as chemical or thermal initiators. Azobisbutyronitrile is a chemical initiator. A combination of a transition metal, especially iron, with a peroxygen and preferably a stabilizing agent such as glucuronic acid allows generation of free radicals to initiate polymerization by a cycling redox reaction.

Combinations of chemical or redox systems with photoinitiated systems have been demonstrated to be effective in WO 96/29370, and are a preferred initiation system for many applications of the macromers of the present invention.

It is also possible to use the macromers with other types of linking reactions. For example, a macromer could be constructed with amine termination, with the amine considered as an active group; and another macromer could be constructed with isocyanate termination, with the isocyanate as the active group. On mixing, the materials will spontaneously react to form a gel. Alternatively, an isocyanate-terminated macromer could be polymerized and crosslinked with a mixture of diamines and triamines. Such a reaction is more difficult to control than a photoinitiated reaction, but could be preferred for high volume extracorporeal production of gels for implantation, perhaps as drug delivery systems. Other pairs of reactants include maleimides with amines or sulfhydryls, or oxiranes with amines, sulfhydryls or hydroxyls.

**Preferred Macromers**

Preferably, the macromers contain between about 0.3% and 20% by weight of dioxanone residues, more preferably, between about 0.5% and 15% dioxanone residues, and most preferably, about 1% to 5% dioxanone residues. In those embodiments where hydroxy acid residues are desired, the macromer contains between about 0.1 and 10 residues per residue of dioxanone, more preferably between about 0.2 and 5, and most preferably one or more such residue per macromer.

In a preferred embodiment, the macromer includes a core, an extension on each end of the core, and an end cap on each extension. The core is a hydrophilic polymer or oligomer; each extension is a biodegradable oligomer comprising one or more dioxanone linkage; and each end cap comprises one or more functional groups capable of cross-linking the macromers. In a particularly preferred embodiment, the core includes hydrophilic poly(ethylene glycol) oligomers with a molecular weight between about 400 and 40,000 Da; each extension includes 1 to 10 residues selected from dioxanone, and optionally further included between one and five hydroxyacid residues, preferably alpha-hydroxy acid residues; wherein the total of all residues in the extensions is sufficiently small to preserve water-solubility of the macromer, being typically less than about 20% of the weight of the macromer, more preferably 10% or less.

Preferably, each end cap includes a polymerizable group. The preferred groups are free-radical (homolytically) polymerizable. More preferably, they are ethylenically-unsaturated (i.e., containing carbon-carbon double bonds), with a preferred molecular weight between about 50 and 300 Da, which are capable of cross-linking and/or polymerizing the macromers. A preferred embodiment incorporates a core consisting of poly(ethylene glycol) oligomers of molecular weight about 25,000 Da; extensions including poly(dioxanone) oligomers with a molecular weight of about 200 to 1000 D, alone or in combination with extensions formed of hydroxy acid oligomers; and end caps consisting of acrylate moieties (which are about 55 Da molecular weight).

## Macromer Synthesis

The macromers can be synthesized using means well known to those of skill in the art. General synthetic methods are found in the literature, for example in U.S. Patent No. 5,410,016 to Hubbell et al., U.S. Patent No. 4,243,775 to Rosensaft et al., and U.S. Patent No. 4,526,938 to Churchill et al.

For example, a polyethylene glycol backbone can be reacted with trimethylene carbonate (TMC) in the presence of a Lewis acid catalyst, such as stannous octoate, to form a TMC-polyethylene glycol terpolymer. The TMC-PEG polymer may optionally be further derivatized with additional degradable groups, such as lactate groups. The terminal hydroxyl groups can then be reacted with acryloyl chloride in the presence of a tertiary amine to end-cap the polymer with acrylate end-groups. Similar coupling chemistry can be employed for macromers containing other water-soluble blocks, biodegradable blocks, and polymerizable groups, particularly those containing hydroxyl groups.

When polyethylene glycol is reacted with TMC and a hydroxy acid in the presence of an acidic catalyst, the reaction can be either simultaneous or sequential. As shown in the examples below, the simultaneous reaction will produce an at least partially random copolymer of the three components. Sequential addition of a hydroxy acid after reaction of the PEG with the TMC will tend to produce an inner copolymer of TMC and one or more PEGs, which will statistically contain more than one PEG residue linked by linkages derived from TMC, with hydroxy acid largely at the ends of the (TMC, PEG) region. There is a tendency for TM groups to re-arrange by "back-biting" during synthesis, which is why multiple PEG molecules can become incorporated in the same macromer. When the hydroxy acid contains a secondary hydroxyl, as in lactic acid, then the tendency towards rearrangement is reduced.

In principle, the degradable blocks or regions could be separately synthesized and then coupled to the backbone regions. In practice, this more complex reaction does not appear to be required to obtain useful materials.

## Sequential Addition

Upon reaction of trimethylene carbonate (TMC) with polyethylene glycol (PEG), the TMC linkages in the resulting copolymers have been shown to form end linked species of PEG, resulting in segmented copolymers, i.e. PEG units coupled by one or more adjacent TMC linkages. The length of the TMC segments can vary, and is believed to exhibit a statistical distribution. Coupling may also be accomplished via the carbonate subunit of TMC. These segmented PEG/TMC copolymers form as a result of transesterification reactions involving the carbonate linkages of the TMC segments during the TMC polymerization process when a PEG diol is used as an initiator. Similar behavior is expected if other polyalkylene glycol initiators were used. The end-linking may begin during the reaction of the TMC with the PEG, and completion of the end linking and attainment of equilibrium is observable by a cessation of increase of the viscosity of the solution.

If the product of this first reaction step is then reacted with a reactive end-capping material, such as acryloyl chloride, a significant percentage of the macromer end groups can be PEG hydroxyls, resulting in the attachment of the reactive groups directly to one end of a non-biodegradable PEG molecule. Such a reaction of the PEG/TMC segmented copolymers can be prevented by adding additional segments of other hydrolyzable co-monomers (e.g. lactate, glycolate, 1,4-dioxanone, dioxepanone, caprolactone) on either end of the PEG/TMC segmented copolymer. Some scrambling of the comonomer segments with the PEG/TMC prepolymer is expected, but this can be minimized by using proper reaction conditions. The basic PEG/TMC segmented copolymer or the further reacted PEG/TMC/comonomer segmented terpolymer is then further reacted to form crosslinkable macromers by affixing reactive end groups (such as acrylates) to provide a macromer with reactive functionality. Subsequent reaction of the end groups in an aqueous environment results in a bioabsorbable hydrogel. Similar segmented structures would be expected if another polyalkylene glycol (PAG) were used, for example a poloxamer.

The copolymers and macromers can have tailorable solubility and solution viscosity properties. The hydrogels can have tailorable modulus and degradation rate. For a given solution concentration in water, the viscosity is affected by the degree of end linking, the length of the TMC (and other hydrophobic species) segments, and the molecular weight of the starting PAG. The modulus of the hydrogel is affected by the molecular weight between crosslinks. The hydrogel degradation rate can be modified by adding a second, more easily hydrolyzed comonomer (e.g. lactate, glycolate, 1,4-dioxanone) as a segment on the ends of the basic PAG/TMC copolymer prior to adding the crosslinkable end group to form the macromer.

Some of these structures described herein are depicted below. PEG, lactate and acrylate units are used solely for purposes of illustration.

SOME BASIC STRUCTURES:

$(CH_2\text{-}CH_2\text{-}O)_x$ = PEG repeat unit=$(PEG)_x$
$(CO\text{-}(CH_2)_3\text{-}O)_y$ = TMC repeat unit=$(TMC)_y$
$(CO\text{-}CH(CH_3)\text{-}O)_z$ = Lactate repeat unit=$(LA)_z$
$\text{-}CO\text{-}CH{=}CH_2$= Acrylate end group=AA

SEGMENTED PEG/TMC COPOLYMER:

$HO\text{-}(CO\text{-}(CH_2)_3\text{-}O)_y\text{-}[(CH_2\text{-}CH_2\text{-}O)_x\text{-}(CO\text{-}(CH_2)_3\text{-}O)_y]_n\text{-}H$
or $HO\text{-}(TMC)y\text{-}[(PEG)x\text{-}(TMC)y]n\text{-}H$

SEGMENTED PEG/TMC/Lactate TERPOLYMER:

$HO\text{-}(CH(CH_3)\text{-}CO)_z\text{-}O\text{-}(CO\text{-}(CH_2)_3\text{-}O)_y\text{-}[(CH_2\text{-}CH_2\text{-}O)_x\text{-}(CO\text{-}(CH_2)_3\text{-}O)_y]_n\text{-}(CO\text{-}CH(CH_3)\text{-}O)_z\text{-}H$
or $HO\text{-}(LA)_z\text{-}(TMC)_y\text{-}[(PEG)_x\text{-}(TMC)_y]_n\text{-}(LA)_z\text{-}H$

SEGMENTED PEG/TMC MACROMER (acrylated):

$CH_2{=}CH\text{-}CO\text{-}O\text{-}(CO\text{-}(CH_2)_3\text{-}O)_y\text{-}[(CH_2\text{-}CH_2\text{-}O)_x\text{-}(CO\text{-}(CH_2)_3\text{-}O)_y]_n\text{-}CO\text{-}CH{=}CH_2$
or $AA\text{-}(TMC)_y\text{-}[(PEG)_x\text{-}(TMC)_y]_n\text{-}AA$

SEGMENTED PEG/TMC/Lactate TERPOLYMER MACROMER (acrylated):

$AA\text{-}(LA)_z\text{-}(TMC)_y\text{-}[(PEG)_x\text{-}(TMC)_y]_n\text{-}(LA)_z\text{-}AA$

**Applications for the Macromers.**

**Methods of Treatment**

Generally, the macromer of the invention may be used in any medical condition which requires a coating or sealing layer may be treated by the methods described herein to produce a coating with better adherence. For example, lung tissue may be sealed against air leakage after surgery using the priming technique. Likewise, wounds may be closed; leakage of blood, serum, urine, cerebrospinal fluid, air, mucus, tears, bowel contents or other bodily fluids may be stopped or minimized; barriers may be applied to prevent post=surgical adhesions, including those of the pelvis and abdomen, pericardium, spinal cord and dura, tendon and tendon sheath. The technique may also be useful for treating exposed skin, in the repair or healing of incisions, abrasions, burns, inflammation, and other conditions requiring application of a coating to the outer surfaces of the body. The technique is also useful for applying coatings to other body surfaces, such as the interior or exterior of hollow organs, including blood vessels. In particlular, restenosis of blood vessels or other passages can be treated. The techniques can also be used for attaching cell-containing matrices, or cells, to tissues, such as meniscus or cartilage.

Sealing Leaks in Tissue

A preferred use of the polymers is in a method of sealing leaks, for example, leaks of gases and/or bodily fluids (such as blood, cerebrospinal fluid, urine and bile), in tissue such as lung , urethra, ureter, gastrointestinal tract, reproductive tract, liver, spleen, dura, and the spinal cord.

In thoracic surgery, uses include sealing of bronchopleural fistulas, reduction of mediastinal bleeding, sealing of esophageal anastomoses, and sealing of pulmonary staple or suture lines. In neurosurgery, uses include dural repairs, microvascular surgery, and peripheral nerve repair. In general surgery, uses include bowel anastomoses, liver resection, biliary duct repair, pancreatic surgery, lymph node resection, reduction of seroma and hematoma formation, endoscopy-induced bleeding, plugging or sealing of trocar incisions, and repair in general trauma, especially in emergency procedures.

In plastic surgery, uses include skin grafts, burns, debridement of eschars, and blepharoplasties (eyelid repair). In otorhinolaryngology (ENT), uses include nasal packing, ossicular chain reconstruction, vocal cord reconstruction and nasal repair. In opthalmology, uses include corneal laceration or ulceration, and retinal detachment. In orthopedic surgery, uses include tendon repair, bone repair, including filling of defects, and meniscus repairs. In gynecology/obstetrics, uses include treatment of myotomies, repair following adhesiolysis, and prevention of adhesions. In urology, sealing and repair of damaged ducts, and treatment after partial nephrectomy are potential uses. Sealing can also be of use in stopping diffuse bleeding in any of a variety of situations, including especially treatment of hemophiliacs. In dental surgery, uses include treatment of periodontal disease and repair after tooth extraction. Repair of incisions made for laparoscopy or other endoscopic procedures, and of other openings made for surgical purposes, are other uses. Additional uses include separation of tissues to prevent damage by rugging during healing. Similar uses can be made in veterinary procedures. In each case, appropriate biologically active components may be included in the sealing or bonding materials.

The method involves priming the surface of the tissue with a polymerization initiator, applying a macromer solution that also contains one or more polymerization initiators to the surface of the tissue to be coated, and then polymerizing the macromer. Preferably, the polymerization initiator comprises a photoinitiator.

Applying the initiator to surface of the tissue before adding the macromer solution polymerizes the macromer at the interface between the solution and the tissue surface. This "interfacial polymerization" provides excellent adherence of the resulting polymer to the tissue surface. Providing an initiator in the macromer solution allows a relatively thick, for example 1 mm to 10 mm, layer of polymer to be formed on the tissue surface. Relatively thick polymer layers may be required to, effectively seal some types of tissue, for example, lung tissue or dura, depending on the size of the leak.

## Prevention of Surgical Adhesions.

Another preferred application of the macromers is in a method of reducing formation of adhesions after a surgical procedure in a patient. The method involves coating damaged tissue surfaces in a patient with an aqueous solution of a light-sensitive free-radical polymerization initiator and a macromer solution as described above. The coated tissue surfaces are exposed to light sufficient to polymerize the macromer. The light-sensitive free-radical polymerization initiator may be a single compound (e.g., 2,2-dimethoxy-2-phenyl acetophenone) or a combination of a dye and a cocatalyst (e.g., ethyl eosin or eosin Y, and triethanolamine).

## Controlled delivery of incorporated agents.

Another preferred use of the macromers involves locally applying an incorporated agent, such as a prophylactic, therapeutic or diagnostic agent, to tissue surfaces of a patient. The method includes the steps of mixing an agent to be incorporated with an aqueous solution including a suitable polymerization initiator, such as a light-sensitive free-radical polymerization initiator, and a macromer, to form a coating mixture. Tissue surfaces are coated with the coating mixture and the macromer is polymerized, for example, by exposure of the coating mixture to an effective amount of light of an appropriate wavelength.

Any of a variety of therapeutic, prophylactic or diagnostic agents can be delivered using these methods. Examples include synthetic and natural inorganic and organic compounds such as proteins (100 amino acid residues or more), peptides (less than 100 amino acid residues), carbohydrates, lipids, nucleic acid molecules, and small synthetic materials such as ethical drugs, having therapeutic, prophylactic or diagnostic activities. Nucleic acid molecules include genes, antisense molecules which bind to complementary DNA to inhibit transcription, aptamers, triple helix forming oligomers and ribozymes. The agents to be delivered can have a variety of biological activities. Diagnostic agents such as radiolabelled compounds, enzymatically labeled compounds, fluorescently labeled compounds, and other detectable agents can also be incorporated. Compounds with a wide range of molecular weights can be incorporated, for example, between 100 and 500,000 grams or more per mole.

Therapeutic or prophylactic compounds ("drugs") of particular interest are those whose efficacy in treatment of a localized medical condition is increased by local delivery of the compound at or near the site of the localized medical condition. Examples of classes of such drugs are those which inhibit the formation or re-formation of scars or adhesions; those which prevent unwanted proliferation of vascular tissue or other luminal tissue; and growth factors, cytokines, etc. which only needs to be effective locally.

Imaging agents which may be utilized include commercially available agents used in positron emission tomography (PET), computer assisted tomography (CAT), single photon emission computerized tomography, x-ray, fluoroscopy, and magnetic resonance imaging (MRI).

Examples of suitable materials for use as contrast agents in MRI include the gadolinium chelates currently available, such as diethylene triamine pentacetic acid (DTPA) and gadopentotate dimeglumine, as well as iron, magnesium, manganese, copper and chromium.

Examples of materials useful for CAT and x-rays include iodine based materials for intravenous administration, such as ionic monomers typified by diatrizoate and iothalamate, non-ionic monomers such as iopamidol, isohexol, and ioversol, non-ionic dimers, such as iotrol and iodixanol, and ionic dimers, for example, ioxagalte.

Hydrogels incorporating these agents can be detected using standard techniques available in the art and commercially available equipment.

The macromers are particularly useful for delivering hydrophilic and/or labile materials. Because the macromer is water-soluble, water can penetrate the polymer and dissolve or extract incorporated hydrophilic materials. Labile materials can be incorporated without exposure of the material to organic solvents which would destroy biological activity. Hydrophobic materials may also be incorporated, if the rate of dissolution of the hydrophobic material and/or the gel matrix is sufficiently rapid to release the material at a therapeutically-effective rate. In all cases, the polymerized hydrogel will tend to protect the therapeutic material from attack by biological activities of the subject, such as enzyme activity.

In a variation of the method for controlled drug delivery, the macromers are polymerized with incorporated

**Preferred Macromers**

Preferably, the macromers contain between about 0.3% and 20% by weight of dioxanone residues, more preferably, between about 0.5% and 15% dioxanone residues, and most preferably, about 1% to 5% dioxanone residues. In those embodiments where hydroxy acid residues are desired, the macromer contains between about 0.1 and 10 residues per residue of dioxanone, more preferably between about 0.2 and 5, and most preferably one or more such residue per macromer.

In a preferred embodiment, the macromer includes a core, an extension on each end of the core, and an end cap on each extension. The core is a hydrophilic polymer or oligomer; each extension is a biodegradable oligomer comprising one or more dioxanone linkage; and each end cap comprises one or more functional groups capable of cross-linking the macromers. In a particularly preferred embodiment, the core includes hydrophilic poly(ethylene glycol) oligomers with a molecular weight between about 400 and 40,000 Da; each extension includes 1 to 10 residues selected from dioxanone, and optionally further includes between one and five hydroxyacid residues, preferably alpha-hydroxy acid residues; wherein the total of all residues in the extensions is sufficiently small to preserve water-solubility of the macromer, being typically less than about 20% of the weight of the macromer, more preferably 10% or less.

Preferably, each end cap includes a polymerizable group. The preferred groups are free-radical (homolytically) polymerizable. More preferably, they are ethylenically-unsaturated (i.e., containing carbon-carbon double bonds), with a preferred molecular weight between about 50 and 300 Da, which are capable of cross-linking and/or polymerizing the macromers. A preferred embodiment incorporates a core consisting of poly(ethylene glycol) oligomers of molecular weight about 25,000 Da; extensions including poly(dioxanone) oligomers with a molecular weight of about 200 to 1000 D, alone or in combination with extensions formed of hydroxy acid oligomers; and end caps consisting of acrylate moieties (which are about 55 Da molecular weight).

**Tissue Adhesives.**

The macromers, and hydrogels formed therefrom, can also be used to adhere tissue surfaces in a patient. The macromer is mixed with a suitable polymerization initiator system, such as a photoinitiator or photoinitiator/amine mixture, to form an aqueous mixture. The mixture is applied to a tissue surface to which tissue adhesion is desired. The tissue surface is contacted with the tissue with which adhesion is desired, forming a tissue junction.. The macromers are then polymerized at the tissue junction.

Such a technique can be used to hold surgically severed tissue in apposition during the healing process, thereby replacing or supplementing the use of sutures, staples, etc. In addition, such a gel may also be used to form a protective barrier.

**Coating of Surfaces**

Surfaces to be coated include biologically-related surfaces of all kinds, and include the surface of drug delivery devices such as catheters or prosthetic implants. Any tissue or cell surface is contemplated, as well as the surface of a device to be used in the body or in contact with bodily fluids. A coating may be applied to the surface of any of these, in an amount effective to improve tenacity of adherence. Moreover, the technique may be used to adhere surfaces to each other. For example, wounds in living tissue may be bonded or sealed using this technique or preformed medical appliances may be bonded to tissue. Examples of such applications are grafts, such as vascular grafts; implants, such as heart valves, pacemakers, artificial corneas, and bone reinforcements; supporting materials, such as meshes used to seal or reconstruct openings; and other tissue-non-tissue interfaces. A particularly important class of tissue surfaces is those which are friable, and therefore will not support sutures well. Adherent coatings can seal the suture lines, support sutured areas against mechanical stress, or substitute entirely for sutures when mechanical stress is low. Examples of such situations include vascular anastomosis, nerve repair, repair of the cornea or the cochlea, and repair of the lung, liver, kidney and spleen.

The priming technique can also be used on non-tissue surfaces in general, where useful bonds may be formed between similar or dissimilar substances, and solid or gel coatings are tightly adhered to surfaces. In particular, a pre-formed gel, or other fragile material, may be tightly adhered to a supporting material by this method.

The priming method is advantageous because it can be used to coat and or to bond together any of a wide variety of surfaces. These include all surfaces of the living body, and surfaces of medical devices, implants, wound dressings and other body-contacting atrificial or natural surfaces. These include, but are not limited to, at least one surface selected from the following: a surface of the respiratory tract, the meninges, the synovial spaces of the body, the peritoneum, the pericardium, the synovia of the tendons and joints, the renal capsule and other serosae, the dermis and epidermis, the site of an anastomosis, a suture, a staple, a puncture, an incision, a laceration, or an apposition of tissue, a ureter or urethra, a bowel, the esophagus , the patella, a tendon or ligament, bone or cartilage, the stomach, the bile duct, the bladder, arteries and veins; and devices such as percutaneous catheters (e.g. central venous catheters), percutaneous cannulae (e.g. for ventricular assist devices), urinary catheters, percutaneous electrical wires, ostomy appliances, electrodes (surface and implanted), and implants including pacemakers, defibrillators and tissue augmentations.

In a particularly preferred application of these macromers, an ultra thin coating is applied to the surface of a tissue, most preferably the inside surface of a blood vessel. Such coatings can be used to treat or prevent stenosis or restenosis of blood vessels. A polymerization initiator, preferably a photoinitiator, is applied to the surface of the tissue, allowed to stain the tissue, and, optionally, the excess photoinitiator is removed by dilution or rinsing. After the initiator has been applied to the tissue, the macromer solution is applied and the macromer is polymerized. As demonstrated below, this method is capable of creating a uniform polymeric coating of between about one and 300 microns in thickness, more preferably about ten to 200 microns, most preferably 20 to 80 microns, which does not evoke thrombosis during its residence at the site.

The surface of medical devices can be coated with the macromers using interfacial polymerization, bulk polymerization, or both, as discussed above. Coating layers applied using interfacial polymerization or a combination of interfacial and bulk polymerization typically adhere more strongly to the medical devices than those prepared using only bulk polymerization.

## APPLICATION TECHNIQUES AND DEVICES

Both priming and polymer addition may be accomplished by simple dripping of material onto the surface to be coated. This can be accomplished using common devices such as a syringe, a pipet, or a hose, depending on scale. More uniform applications may be obtained using an applicator, such as a brush, a pad, a sponge, a cloth, or a spreading device such as a finger, a coating blade, a balloon, or a skimming device. These may further be used to rub the surface to improve penetration of the primer or the monomer, or to mix primer and monomer *in situ* on the surface. In large-scale applications, fluid layers may be applied with large-scale coating machinery, including roll coaters, curtain coaters, gravure and reverse gravure devices, and any of the coating devices known in the art. Sprayers may be used at any scale, especially for lower-viscosity primers or polymerizable monomer layers.

Application techniques and devices may be combined, as in applying fluid from a syringe, and then rubbing it into the surface with a finger tip. Such operations may be repeated, as in applying drops of priming initiator; rubbing these into the surface with a brush; repeating this operation; adding monomer solution; rubbing it in; and finally applying additional layers of monomer before or during the application of curing means, such as light, heat, or slow release of peroxide radicals.

An additional application means which is required in many coating techniques described herein, and in particular in the preferred coating method which uses photoinitiation to cure the monomer, is a light source. For large-scale application, flood lamps and similar devices are useful. In small, localized applications, such as tissue sealing and coating, it may be preferable to use a localized source such as a fiber optic or light guide, which can project radiation of the appropriate wavelength onto the site to be treated to cause polymerization of the monomer. Also, a light emitter could be carried on a device, as a miniature bulb. A focused beam from a remote source could be suitable if, for example, the surface was exposed. In exposed surfaces, it is possible that ambient light could be sufficient to polymerize the coating, especially at high initiator levels.

Each of the applications means can be separate, so that a kit of application means could contain, for example, one or more containers or reservoirs, one or more pads or brushes, and if required at least one light guide. The application means could also be combined in whole or in part. For example, a dripping device, such as a tube, could be combined with a spreading device, such as a brush. These could further be combined with a light guide. Such combination devices are especially desirable in treatment of living organisms, and especially humans, to maximize the simplicity of a procedure and the probability of correctly conducting it.

COMPLIANCE PROPERTIES

The compliance properties of the material herein described are those of the material after it has polymerized to form a polymerized material. As used herein, "polymerized material" includes material which forms by the ionic or covalent reaction of monomer precurser molecules. Preferably, the polymerized material is formed by covalent reactions of the monomers. It can be very difficult to measure the elastic properties of the material when adhered to tissue. The mechanical properties are therefore when appropriate measured on samples made *in vitro,* either in a mold, or, as in the lap-shear test, in contact with standardized tissue. Such measurements must be corrected to conditions applicable to tissue treatment, including the diluting effects of polymerization reagents, or of fluids on the tissue. Thus, a sealing solution may be applied to tissue at a concentration of 30%, but in the coating process it may be diluted to 15% effective concentration by dilution with blood or plasma. Similarly, especially in the case of fibrin sealant, the polymer concentration may be reduced by mixing with polymerizing reagents, either in bulk or by spraying. Where appropriate, such corrections have been taken into account in the descriptions herein. Materials may be equilibrated with water before testing either by absorption or syneresis.

In light of these observations, an effective material for forming a compliant coating or sealant preferably has a strain or elongation before fracture substantially similar to or at least as great as the expected strain during normal use of the tissue to which it is applied, and the elongation of the polymerized material is preferably reversible. This is to avoid either detachment from the tissue or fracture, or limitation of the tissue's natural expansion. Preferably, the effective compliant material will have a reversible elongation at least about 150% as great, more preferably at least about 200% as great, and still more preferably at least about 300% as great as the expected strain of the tissue.

The polymerized material thus may be designed and selected for application to different tissue, to have an elongation at rupture which is similar to or greater than the elongation of the tissue in vivo during its function. The elongation at rupture of the polymerized material can be, for example, greater than 100% or 200%, or optionally greater than 300% or 400%. In some embodiments, the elongation at rupture of the polymerized material may be between for example 100% and 700%, depending on the tissue properties. In some applications, an elongation at rupture greater than 700% is useful.

In addition, the compliant material, for example in sealant applications, preferably should have a normalized compliance that is comparable in magnitude to the normalized compliance of the tissue to which it is applied. The material will be operative even when the material's normalized compliance is much greater than the normalized compliance of the tissue.

In cases where minimal modification of the natural expansion and contraction of a tissue is desired, the preferred range of the normalized compliance ratio extends from about 0.05 to about 3, preferably from about 0.1 to about 2.0, and more preferably from about 0.1 to about 1.0. In some cases, for example when the tissue is lung tissue, a value of the elastic modulus of less than about 150 kPa, preferably less than 100 kPa, more preferably less than about 50 kPa, and most preferably less than about 30 kPa is preferred.

To obtain the desired ratio of the normalized compliance of the polymerized material to the normalized compliance of tissue, the overall force required to stretch the sealant layer should be adjusted, since that of the tissue is fixed. The adjustment can be accomplished by any of several known methods, including the alteration of the thickness of the layer of the polymerized material, or the variation of the polymer concentration, or of the polymer crosslink density, or of other properties of the material. The properties of the precursor materials and the reaction conditions may be adjusted to produce desired other properties of the polymerized material, such as sealant or adhesive properties, or controlled degradation and drug release properties.

Where prevention of tissue deformation is desired, for example during a healing period, the parameters of the tissue coating can be adjusted so that the normalized compliance ratio is significantly in excess of 1.

The adherence of the polymerized material to the tissue is important in order to obtain the benefits of proper compliance properties. An adherence of at least about 20 gm/cm$^2$ in a single or double lap shear test is preferable for many applications. Use of priming technology, described elsewhere in this application, is an effective method for obtaining such values. In some applications, such as the use of the polymerized material as a tissue sealant, adherence values of about 30 gm/cm$^2$ are preferred, and values at or above 40 gm/cm$^2$ are more preferred.

In many applications, such as tissue sealing, the viscosity of the precursor materials can be tailored to obtain optimal coatings. Higher viscosities can favor retention of the uncured or unpolymerized sealant at the site of application, and minimize displacement of the sealant by the presence of bodily fluids at the surface. However, higher viscosities make the material more difficult to apply. A suitable range of viscosity, for example, for the sealant portion of a sealing system is in the range of about 200 cP (centipoise) to about 40,000, preferably about 500 to about 5000 cP, and more preferably about 700 to about 1200 cP. For lung, a suitable range of viscosity is about 900 to 1000 cP. The optimal viscosity will depend on the site of application and the nature of the condition which is to be alleviated by the application of the material.

The present invention will be more fully understood by reference to the following non-limiting examples.

### Example 1: General Synthesis of Macromers: Melt Method.

Methods analogous to those described in U.S. Patent No. 4,526,938 to Churchill et al. were used to form derivatized PEG by the melt method. Polyethylene glycol (PEG) was obtained commercially. The molecular weight listed on the label was assumed to be the molecular weight of the material. The PEG was optionally dissolved in methanol and purified by passage over an ion exchange resin, and dried.

Purified or as-supplied PEG was charged to a reactor, optionally with a small amount of xylene, and heated for five to six hours at about 110°C (note: all temperatures herein are in degrees Celsius) under vacuum to complete removal of water. After cooling under vacuum, the flask was placed in a glove bag, the materials for forming the biodegradable linkages (including T (trimethylene carbonate) and L (lactide)) were added to the PEG, and the temperature was raised to about 160-165°C under an argon blanket.

After dissolution of the reactants in the melted PEG, a catalyst, typically stannous octoate, was added, the temperature was raised to 185°C, and ring-opening addition was allowed to proceed for about 3 hours, with stirring under argon. The PEG-(T,L) intermediate could be further reacted at this stage, but typically was freed of unreacted monomers by precipitation in hexane, recovery and drying.

The purified intermediate, for example, PEG-(TnLm), where "n" is the number of T groups and "m" is the number of L groups, or the original reaction mixture without purification, was taken up in toluene, and an agent capable of adding unsaturated linkages, such as acryloyl chloride, was added, typically in excess, under mild heating (e.g., about 50°C) and in the presence of an acid-neutralizing agent such as triethylamine. Suitable reactant ratios were found to be 1 ml acryloyl chloride and 1.8 ml triethylamine per 30 grams of PEG. The endcapped macromer, PEG-(T,L)-A2, was purified by precipitation in hexane, recovered and dried. Stabilizer was optionally added at this stage. The extent of incorporation of monomers was determined by NMR.

A similar procedure was used to prepare other macromers. PEG-(Tn)-A2, PEG-(Dn, Gm)-A2, where D is dioxanone and G is glycolide, and like materials were formed by similar procedures. Synthesis of macromers based on other starting hydrophilic blocks follows similar procedures, with adjustment in precipitation conditions as required. In a synthesis with multi-hydroxy compounds, such as polyvinyl alcohol, the water would be removed azeotropically under mild reflux in, e.g., toluene; and the degradable linkages are preferably synthesized by polymerization onto the hydroxy compound as described above, although such blocks could be added as preformed activated acrylated blocks, for example, using a carbodiimide derivative of the acylated poly(degradable linkage), if greater control were required.

### Example 2: Synthesis of PEG-TMC and PEG-TMC-Lactide Macromers.

35K(T8)A2 ("35KT" in the examples below) was made from purified PEG of nominal molecular weight 35,000 Daltons by the melt procedure as described above. T was charged into the reactor at a nominal molar ratio of 13:1 to the PEG to obtain this result. The final actual acrylate incorporation averaged 1.6 per PEG molecule, or about 2 acrylates per macromer.

35K(T7L2)A2 ("35KTL") contained about 7 T units (6.88 measured) and 2 lactate units (1.86 measured) as synthesized. (Note that there are 2 lactate units per lactide molecule.) T and L were charged at nominal molar ratios of 10:1 and 3:1 relative to PEG.

20K(T30L 15)A2 contained about 30 T units and 15 lactide units per 20,000 Daltons PEG molecule. The actual acrylate to PEG ratio was 1.42.

### Example 3: Seal pressure test on latex to measure strength and elasticity

Poly(ethylene glycol)-lactide-trimethylene carbonate terpolymers endcapped with acrylate esters were evaluated using a seal pressure test apparatus to determine the failure pressure for coatings prepared using the macromers.

One of the materials tested had a poly(ethylene glycol) molecular weight of 20,000 Daltons ("20 kiloDaltons"), a lactate incorporation of 13.8 and a trimethylene carbonate incorporation of 16.0, with nominal acrylation of 2 per macromer ("20KTL"). Also tested were 35 kiloDalton PEG esterified with about 8 TMC linkages and then endcapped with acrylates ("35KT"), and 35 kiloDalton PEG esterified with about 8 TMC and about 8 lactide groups ("35 KTL"), both also acrylated. The reagents applied were "primer" and "sealant". The complete system contained both a photoinitiation system (Eosin Y/triethanolamine) and a redox initiation system (ferrous gluconate/fructose, plus t-butylhydroperoxide after mixing) which did not cause significant polymerization of the macromer until both mixed (primer and sealant) and activated by light.

Primer solution contained Eosin Y (2000 ppm, w/w), 5000 ppm ferrous gluconate, 10,000 ppm (1%) fructose, 30,000 ppm NaCl, and 30% w/w of 3.5KL5 macromer (made according to US 5,410,016). Primer was applied to a 2 cm x 2 cm piece of latex film (from a latex examination glove; about 1 mm thick) with a 6 mm diameter hole created in the center. A toroidal Teflon® fluoropolymer template with a 1 cm. diameter central hole was placed on the latex film to control the area of application of the sealant, by limiting its spread.

Sealant solution contained macromer (in this example, 10% or 20% w/w of one of the macromers described above) dissolved in an aqueous solution containing isotonic saline, 90 mM triethanolamine as buffer and electron transfer component, Eosin Y (20 ppm) as a photoinitiator, 4000 ppm vinylcaprolactam as comonomer, and 125 ppm t-butylhydroperoxide as part of the light-sensitized redox initiation system. Two drops of sealant were dispensed inside the template above the hole and then carefully mixed in with the primer, using a brush. Three more drops of sealant were dispensed, and then the area was illuminated with visible light from a xenon arc lamp (450-550 nm) at an intensity of 100 mW/cm$^2$ for 40 seconds. Samples were placed in phosphate buffered saline (pH 7.4) at 37°C for different times (t=0, 4 hours, 1 days, 3 days, 6 days and 10 days).

Samples were evaluated at each time point using a seal pressure test apparatus to determine the failure pressure. The test apparatus was a modified membrane holder, in which the latex sample was clamped at the edges between gaskets. Pressure was then applied to the side of the latex away from the polymer seal, and the pressure required to rupture the seal was measured.

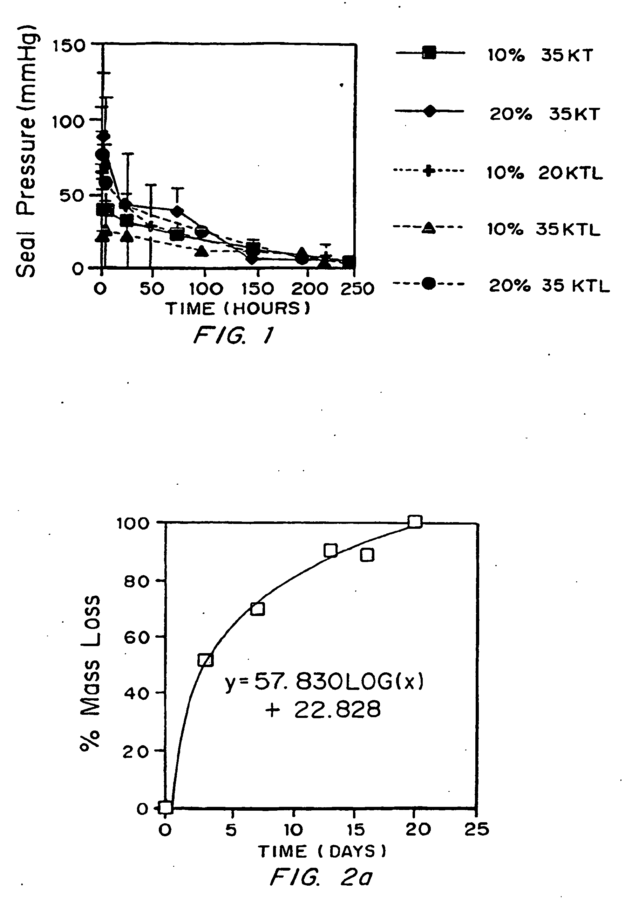

Figure 1 shows the results obtained with five different sealant materials. The seal pressure decreases over the course of time, presumably due to a combination of swelling of the hydrogel due to hydration and programmed degradation of the hydrogel.

## Example 4: Bioabsorption in-vivo

Poly(ethylene glycol)-trimethylene carbonate copolymers, optionally containing lactate, and endcapped with acrylate esters, were evaluated. Two of the materials described in Example 3 were used (20KTL and 35KT). The macromers were polymerized using visible light illumination from a xenon arc lamp (450-550 nm) at an intensity of 100 mW/cm$^2$ in a sealant solution containing macromer, eosin, triethanolamine, vinylcaprolactam, t-butylhydroperoxide and saline as in Example 3. Macromer concentration was 10% for 20KTL and 20% for 35KT.

The absorption of the sealant was determined by subcutaneous implants in rats. Five female Sprague Dawley rats (250-300 g) were used in this study. The animals were anesthetized by intramuscular ("IM") injection of 3.2 ml/kg mixture of Ketamine (52.4 mg/kg), Xylazine (2.8 mg/kg), Acepromazine (0.7 mg/kg). Four 1 cm longitudinal incisions were made through the skin on the back. Two incisions were made on each side of the spinal column, spaced 1 cm off the midline and 2 cm apart. A 2 X 2 cm pocket was created at each incision site by blunt dissection. Preformed hydrogel disks were prepared using sterile technique. One hundred microliters of sealant solution was placed in the bottom of one well of a standard 24-well tissue culture plate, and was illuminated for 40 sec at 100 mW/cm$^2$, producing a thin disc about 18 mm. in diameter. One disk was placed in each subcutaneous pocket. Incisions were then closed with surgical staples. Animals were euthanized at intervals by $CO_2$ inhalation. Incisions were opened and gross observations were recorded. Each site was harvested and analyzed by gel permeation chromatography for mass loss.

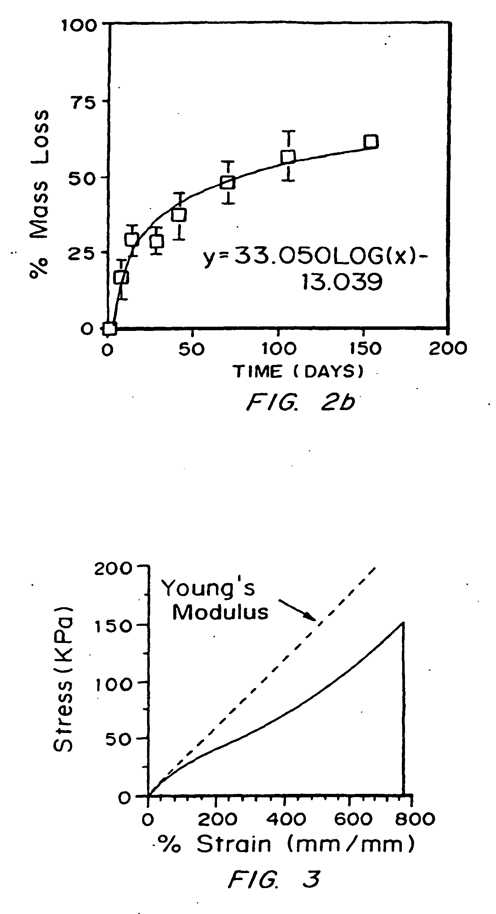

Figures 2A and 2B show that the 20KTL based hydrogels were completely absorbed in 20 days *in vivo* (Figure 2A), whereas the 35KT based hydrogels were partially absorbed (60% weight loss) in 154 days (Figure 2B). This illustrates the significant effect of rapidly-degrading linkages such as lactate groups.

## Example 5: Sealing of Dural Leak in Canine Craniotomy

A poly(ethylene glycol)-trimethylene carbonate-lactate copolymer endcapped with acrylate ester (20KTL, from Example 2) was evaluated for its ability to seal fluid leaks in membranes *in vivo.* The poly(ethylene glycol) molecular weight used was 20,000 Da. The lactate incorporation was 13.8 and the trimethylene carbonate incorporation was 16.0. The macromer was fully acrylated.

The performance of the sealant was evaluated in a dural incision injury model in three mongrel canines. The animals were pre-anesthetized, intubated and maintained on isoflurane gas anesthesia. With the head elevated, a bilateral craniotomy was created. Two dural incisions, each 2 cm. long, were made, one on the left side and one on the right side. Incisions were closed using 3 simple interrupted 4-0 to 6-0 silk sutures spaced approximately 5 mm apart. Cerebrospinal fluid (CSF) pressure was raised by inflating the lungs to 20 cm $H_2O$, and the leak was assessed. All incisions were found to leak, as expected from the closure.

The control side (left) received no additional closure of the incision. For the treatment side (right), the primer (a low-viscosity solution; described in example 4) was applied and mixed with the fluid on the tissue interface. The sealant (10% w/w solution of 20KTL (described above) with other materials as in Example 4) was layered over the primer, and the primer and sealant were mixed with a brush. Illumination for 40 sec. with a visible xenon arc lamp (450-550 nm) at an intensity of 100 mW/cm$^2$ was then performed to complete the polymerization.

The leaks were re-verified by raising the CSF pressure by inflating the lungs to 20 cm $H_2O$. Leakage was observed on the control side, but not on the experimental side. The bone flaps were replaced and the incision was repaired.

All animals were euthanized at 21 days and the craniotomy was re-opened. Adhesions between the dura and the bone flap were recorded, and the dura repair sites were inspected for evidence of CSF fluid leaks. Dural repair sites were dissected free from underlying cortical tissue and the presence of adhesions noted. The tissues were harvested, rinsed in saline, and fixed in 10% neutral buffered formalin. Hematoxylin and eosin stained histological sections were taken.

At the time of explant, the control sites showed a slight fibrosis present between the dura mater and calvarium. No inflammation was detected. The sealant was not apparent in two out of three animals and only small amounts were present in the third animal. The dural edges were approximated. Defects in the bone (resulting from the surgery) were filled with fibro-osseous tissue at 21 days, in both control and treated sites. Normal healing appeared to be taking place, both visually and histologically.

### EXAMPLE 6. Solution Synthesis

Macromers can also be made by synthesis in solution. While requiring additional waste disposal, the solution synthesis is more easily controlled (compared to the melt synthesis of Example 1 or 2), and is preferred for most applications. This example also illustrates the practice of sequential addition of monomers; the method is also useful for simultaneous addition of monomers.

35KTLA was made by dissolving 75 g of 35 kiloDalton (nominal) PEG in toluene to 20% w/w concentration, and was dried by nitrogen purge for 3 hours at 108° C. 3.06 G TMC (14 mole equivalents per PEG) and 0.024 g stannous octoate catalyst were added, and the solution was held at 108° for 4 hours with stirring. Then 0.46 g (1.5 equiv..) lactide was added, and stirring continued for 2 hrs. Then 300 ml toluene was added to give about a 10% wt/vol solution of polymer. After cooling the solution to about 50°, 4.6 g of triethylamine and 2.5 ml of acryloyl chloride were added. The solution was stirred for 20 min. at about 50. Acrylated macromer was recovered by precipitation and optionally filtration as described in Ex. 1 and 2. The resulting polymer contained 5 TMC residues and 1.2 lactate residues per PEG molecule, and ratios of lactate :acrylate of 0.6.

### EXAMPLE 7. In Vitro Degradation

35KTLA2 was synthesized as described in Example 6. The completed macromer had a TMC:PEG molar ratio of 3.57; an acrylate: PEG ratio of 1.52; a lactate: PEG ratio of 1.39; and contained 766 ppm TMC. On gel permeation chromatography, the macromer was 48.1 % "monomer" (one PEG unit per macromer molecule), 46.3 % "dimer" two PEG units per macromer molecule), 5.4% "trimer" and 0.1 % higher oligomer.

Discs of the macromer were made as described in example 4 and were incubated in phosphate-buffered saline, pH 7.4, at 37° and 57°. At 57°, half of the mass was lost at about 140 hrs, while at 37°, half the mass was lost at about 42 days. Mass loss was determined by rinsing the specimen, drying to constant weight, and correcting for the amount of buffer and salt present.

### EXAMPLE 8. Dioxanone-containing Macromers

Dioxanone (1,4-dioxan-2-one; p-dioxanone) was synthesized from DEG (diethylene glycol) essentially according to US 2,807,629, Example 1. Two kilograms of DEG were mixed with 40 g. copper chromite (Aldrich) and heated at about 230° for 4 hours under nitrogen purge, which displaced generated hydrogen. Dioxanone and DEG were distilled from the mixture under vacuum at about 50° pot temperature. DEG was partially removed by extraction in cold (4°) diethyl ether. The partially purified material was dissolved in chloroform and applied to a silica column equilibrated in chloroform. Dioxanone was recovered in the initial fractions.

Macromer 35KDA was made by drying overnight (vacuum, 110°) 15 g of 35 kiloDalton PEG, 1.7 g dioxanone, and 0.01 g of catalyst (stannous octoate). This is a ratio of 21:1 D:PEG. Samples were also made at other amounts of D (1.0 g, 18:1 ; 0.84 g, 15:1; 1.34 g, 24:1). Vials were sealed and heated at 150 for 5 hrs. Samples were dissolved in chloroform; optionally precipitated in ether; and acrylated as described in previous examples. NMR showed final molar ratios in the synthesized polymer of D/PEG, 1.82; Ac/PEG, 1.64.

Macromer 35KDLA: To a mixture of 15 g PEG, 0.84 g Dioxanone and 0.01 g catalyst was also added 0.39 g d,1-lactide. The mixture was dried, then incubated at 185 ° overnight; dissolved in chloroform and precipitated in ether, filtered and vacuum-dried; and acrylated with excess acryloyl chloride and triethylamine.

In the following Examples 9-10, the following methods and parameters were used. A person of skill in the art can also look to PCT WO 96/29370 for other techniques that may be applicable.

Elongation to fracture and Young's or other elastic modulus.

Samples are prepared in a mold to have the required concentration of monomer and other ingredients. The crosslinked or otherwise cured specimens are placed in an appropriate machine, such as an Instron™ tester, and the force required to stretch the sample along a single axis is measured as a function of the distance the sample is stretched (strain). Elongation may be continued until the sample breaks, giving the value for elongation at break, optionally after cycling at lower elongations to determine the degree of any plastic deformation of the sample. The data (force vs. distance) may be recorded and used to make a plot, as in Figure 3. Because the response of a particular material is not necessarily "ideal", especially at high elongation, a modulus may be calculated from values at low degrees of elongation where the behavior is closer to linear. Alternatively, the force vs. strain values may be used directly without extrapolation, or without division by sample thickness to give the "normalized compliance" discussed above.

Bulk Compression Modulus.

The sample of gel or tissue is placed in a suitable instrument, such as a Perkin-Elmer DMA 7e, and the modulus is measured according to a standard procedure. A gel sample could also be polymerized directly in the instrument for testing.

Adhesive strength.

This was tested by a lap shear test. The test sealant material was used to adhere a 1 cm x 1 cm area of two pieces of test substrate, typically a standardized tissue such as rat peritoneum or pig pericardium. After crosslinking or curing of the test material, the force required to break the adhesive bond was determined using a suitable instrument, such as an Instron™ tester. In one variant of the test, three pieces of substrate were adhered: a center piece, with tab extending in one direction, and a pair of outer pieces with tabs extending in the opposite direction; sealant was used to join all three pieces. Either arrangment also can be used to determine the relative mechanical properties of various samples (i.e., compared to standards) at small displacements, which is useful when only limited sample volume is available.

Adherence

Adherence of sealant formulae *in vivo* is determined qualitatively, by the relative resistance of the sealant to displacement from its deposition site by a probe.

Viscosity

Viscosity was measured by standard methods, typically in a Brookfield™ viscometer.

Seal Pressure Testing

Seal Pressure Testing was performed by punching a 3 mm round hole in a standard tissue, such as pig pericardium, and mounting the tissue as the closure in a test fixture. Sealant was applied to the hole and cured, typically in a spiral pattern, to obtain closure of the hole. Then increasing pressure was applied to the transverse side of the tissue until the plug of sealant was displaced.

Sealant Polymerization

In Examples 9-10 below, a preferred formulation of the sealing system was used. When applied to tissue or to a surface to which adherence was required, the surface was primed with a mixture which contained by weight approximately 65% water, 30.4% of a polymerizable macromer (3.3KL5A2, a 3.5 kD polyethylene glycol backbone carrying an average of 5 lactate groups and end capped with acrylate), 3% NaCl, 1% fructose, 0.5% ferrous gluconate, and 0.2% Eosin Y. The primer was applied to the surface and spread with a brush. Then about 2 volumes of sealant solution was applied and mixed with a brush. The sealant contained about 77% water, 20.5% polymerizable macromer 35KTMC8A2 (35 kD polyethylene glycol carrying an average of 8 trimethylenecarbonate groups and end capped with acrylate), 1.1% triethanolamine, 1%KH2PO4, 0.4% vinylcaprolactam, 0.013% t-butyl hydroperoxide, and 0.002% Eosin Y. When the sealant solution was tested in isolation, the t-butyl hydroperoxide was omitted. The sealant system was photopolymerized by exposure to blue-green light for about 40 sec.

**EXAMPLE 9. Elasticity Results.**

Using the materials described above, lap shear testing samples were prepared by applying the macromer solution with a cotton swab to a 1 cm X 1 cm area on a 3 cm X 1 cm strip of rat peritoneal tissue, then laying the other strip of the same size on top as to make a sandwich. The sample was then transilluminated from the top and then the bottom for 40 sec each. Lap shear testing was performed using a 12.5 nun gauge length. Tensile testing using a sample size of 45 mm X 10 mm X 5 mm and a 12.5 mm gauge length, was performed. DMA (Perkin Elmer) testing with a sample height of 1.6 mm was performed at 37°C after hydrating for 2 hours in saline at 37°C. A subjective scoring system was used to assess adherence in a goat lung model on a scale from 1-4 (1 = poor adherence & 4 = excellent adherence).

In Vitro Testing

This synthetic surgical sealant could be rapidly polymerized with visible light to form a flexible hydrogel. As can be seen from the tensile data in Figure 3, this material showed a completely elastic deformation profile with linear elongation at break in excess of 700%. The polymerization process of this material and the properties of lung tissue and muscle tissue were studied using the dynamic mechanical tester. It was seen that muscle tissue, as expected, had a higher modulus than spongy parenchymal lung tissue. The sealant material was cured within 40 seconds and reached a final modulus very comparable to that of the lung tissue. This ensures a compliant and persistent adhesive bond. The bond strength was determined using the lap shear test apparatus and the material was seen to form a strong yet flexible bond to tissue. This bond strength is in excess of literature values for fibrin glues in comparable tests.

In Vivo Testing

All goats that had undergone the thoracotomy procedure survived the surgery uneventfully. Goats were sacrificed at timepoints of 14 days, 1 month, and 3 months. At all timepoints, the hydrogel was seen to be firm and clear and had an adherence score of 3.0-3.5 out of 4.0. No tissue necrosis was evident. Histological sections of the tissue showed normal healing. The results are shown below in Table 1.

Table 1: In-Vitro Testing Summary

**Property; Result**

Compressive modulus at full cure, sealant; 32.4 kPa

Compressive modulus of lung tissue, pig; 27.5 $\pm$ 3.4 kPa

Compressive modulus of lung tissue, dog; 28.0 $\pm$ 1.9 kPa

Modulus of rat muscle tissue; 73.4 $\pm$ 6.8 kPa

Young's modulus at full cure, sealant; 29.4 kPa

Elongation at break, sealant; 788 $\pm$ 255.2 %

Sealant lap shear strength; 90.17 $\pm$ 18.17 g/cm$^2$

**EXAMPLE 10. Comparative Results.**

Tissucol™ sealant is a commercial fibrin sealant used in Europe. It is not at present approved for use in the United States, in part because it is made from human serum and thus may carry infectious agents. Tissucol sealant was used according to its manufacturer's directions. In comparison to the preferred sealant formulation of the previous example, the following results were obtained shown in Table 2:

Table 2: Properties of Sealants

| Test: | FocalSeal™ Sealant | Tissucol™ Sealant |
|---|---|---|
| A. Double lap shear | 38 $\pm$ 6 kPa | 10 $\pm$ 6 kPa |
| B. Compression Modulus | 32 $\pm$ 1 kPa | 35 $\pm$ 5 kPa |
| C. Viscosity | ≈780 cP at 20% conc. | 117 cP (fibrinogen) 1.6 CP (thrombin) |

(continued)

| Test: | FocalSeal™ | Tissucol™ |
|---|---|---|
| | Sealant | Sealant |
| D. Seal Pressure Test | ≈380 ± 100 mm Hg | ≈30 ± 20 mm Hg |

When applied to a living dog lung, the fibrin sealant had an adhesion score of 1, and leaked on all staple lines at 10-40 mm Hg. It was difficult to apply the fibrin material to a punch-type leak, because air bubbles coming through the leak tended to remove the material before it polymerized. In contrast, sealant adhered to primed tissue with an adhesion of 3.5, and typically withstood 80 mm Hg or more of pressure. Its high viscosity slowed bubble penetration.

The optimal material for lung, as described above, has an elongation at break of over 700%. Other materials were suitable, if less optimal. For non-collapsed lung, a material (20KT8A2) with an elongation at break of 225% was suitable, while a material (8KL5A2) with an elongation at break of 100% (and an elastic modulus of 47 ±4 kPa) was not effective in lung. The expansion of a dog lung was measured. It was found that the effective area expansion during a normal breathing cycle is about 200%, while the expansion from the atalectatic (collapsed) state to full inflation changed area by about 300%. In the latter case, an extension (strain) of about 100% was observed along one axis, and about 200% along a perpendicular axis, implying non-uniformity of the tissue structure.

Thus, an important requirement for a sealant system on this tissue appears to be that the normalized compliance of the sealant is greater than the normalized compliance of the tissue to which it is applied. While the lung is perhaps the most dramatic example of tissue elasticity and area expansion during normal physiological processes, other tissues, such as the bowels, the bladder and large arteries, can change surface area substantially during normal physiological cycles. Other tissues, such as the beating heart, exhibit significant changes in shape (shear) without necessarily changing local area.

The compliance of the sealant may be selected depending on the tissue to which it is to be applied. A sealant having a high value of normalized compliance, or a low value of the normalized compliance ratio (tissue /material), may be beneficial for certain applications. For example, the 700% -elongation low-modulus material described above is also suitable for sealing the dura of the brain, or the spinal cord after laminectomy, even though these tissues are relatively non-compliant (i.e., are difficult to stretch). Thus, high normalized compliance sealant appears to be useful on most tissues, and desirable as a material having a broad range of applications.

## Claims

1. A biodegradable, polymerizable macromer having a solubility of at least one gram/liter in an aqueous solution at a temperature in the range between 0 and 50°C comprising at least one water-soluble region, at least one biodegradable region, and at least one reactive polymerizable group or polymerizable region capable of crosslinking with other macromers, wherein the polymerizable regions are separated from each other by at least one biodegradable region, and wherein at least one biodegradable region comprises a trimethylene carbonate or dioxanone linkage.

2. The macromer of Claim 1 wherein the water-soluble region is attached to a biodegradable region and at least one polymerizable group or region is attached to the biodegradable region.

3. The macromer of Claim 1 wherein the water soluble region forms a central core, at least two biodegradable regions are attached to the core, and at least two polymerizable groups or regions are attached to the biodegradable regions.

4. The macromer of Claim 1 wherein the biodegradable region is a central core, and at least one polymerizable group or region is attached to the core so that each polymerizable group or region is separated from each other polymerizable group or region by at least one biodegradable region.

5. The macromer of Claim 1 wherein the water soluble region is a macromer backbone, the biodegradable region is a branch or graft attached to the macromer backbone, and at least two polymerizable groups or regions are attached to the biodegradable regions.

6. The macromer of Claim 4 having two or more polymerizable groups or regions attached to the core.

7. The macromer of Claim 1 wherein the water soluble region is a star backbone, the biodegradable region is a branch or graft attached to the water soluble star backbone, and at least two polymerizable groups or regions are attached to one or more biodegradable branch or graft, so that each polymerizable group or region is separated from each other polymerizable group or region by at least one biodegradable region.

8. The macromer of Claim 1 wherein the biodegradable region is a star backbone, the water soluble region is a branch or graft attached to the biodegradable star backbone, and two or more polymerizable groups are attached to the biodegradable backbone so that each polymerizable group or region is separated from each other polymerizable group or region by at least one biodegradable region.

9. The macromer of Claim 1 wherein the water-soluble region is also the biodegradable region.

10. The macromer of Claim 1 wherein the water soluble region is also the biodegradable region, and one or more additional biodegradable regions are grafts or branches upon the water soluble region.

11. The macromer of Claim 1 comprising a water-soluble core, at least two biodegradable extensions on the core, and an end cap on at least two extensions,
   wherein
   the core comprises poly(ethylene glycol);
   at least one of the extensions comprises a biodegradable trimethylene carbonate or dioxanone linkage; and
   each end cap comprises a group or region which is polymerizable by a free radical reaction.

12. The macromer of Claim 11 wherein at least one of the extensions comprises a biodegradable poly(hydroxy acid).

13. The macromer of Claim 12 wherein the poly(ethylene glycol) has a molecular weight between 400 and 40,000 Da; the poly(hydroxy acid) oligomers have a molecular weight between 200 and 2000 Da; and the polymerizable group or region has a molecular weight between 50 and 200 Da.

14. The macromer of Claim 13 wherein the poly(ethylene glycol) oligomers have a molecular weight of about 20,000 Da; the poly(hydroxy acid) oligomers have a molecular weight about 1000 Da; and the polymerizable groups have a molecular weight of about 50 Da.

15. The macromer of Claim 1 wherein the polymerizable group contains a carbon-carbon double bond capable of cross-linking and polymerizing macromers.

16. The macromer of Claim 1 wherein crosslinking and polymerization of the macromer are initiated by a light-sensitive free-radical polymerization Initiator with or without a cocatalyst, further comprising a free radical polymerization initiator.

17. The macromer of Claim 16 wherein the initiator is selected from the group consisting of xanthine dyes, acridine dyes, thiazine dyes, phenazine dyes, camphorquinone dyes, and acetophenone dyes.

18. The macromer of Claim 17 wherein the initiator is selected from the group consisting of eosin, ethyl eosin, 2,2-dimethyl-2-phenyl acetophenone, and 2-methoxy-2-phenyl acetophenone.

19. The macromer of Claim 1 wherein crosslinking or polymerizations are initiated *in situ* by light having a wavelength of 320 nm or longer.

20. The macromer of Claim 1 wherein at least one biodegradable region is selected from the group consisting of poly(alpha-hydroxy acids), poly(lactones), poly(amino acids), poly(anhydrides), poly(orthoesters), and poly(phosphoesters).

21. The macromer of Claim 20 wherein the poly(alpha-hydroxy acid) is selected from the group consisting of poly(glycolic acid), poly(D,L-lactic acid) and poly(L-lactic acid).

22. The macromer of Claim 20 wherein the poly(lactone) is selected from the group consisting of poly(epsflon-caprolactone), poly(delta-valerolactone) and poly(gamma-butyrolactone).

23. The macromer of Claim 1 wherein the water soluble region is selected from the group consisting of poly(ethylene glycol), poly(ethylene oxide), poly(vinyl alcohol), poly(vinylpyrrolidone), poly(ethyloxazoline), poly(ethylene oxide)-co-poly(propylene oxide) block copolymers, polysaccharides, carbohydrates, proteins, and combinations thereof.

24. The macromer of Claim 1 further comprising a prophylactic, therapeutic or diagnostic agent.

25. A solution of a biodegradable, polymerizable macromer as claimed in any one of Claims 1 to 24 for use in medicine.

26. The use of a solution of a macromer as defined in Claim 25 for the manufacture of a medicament for forming a biocompatible coating on tissue, wherein tha tissue surface comprises a polymerization initiator capable of initiating polymerization via free radical or cationic polymerization.

27. A method for making a device for controlled release of a prophylactic, therapeutic or diagnostic agent comprising:

   a) mixing a prophylactic, therapeutic or diagnostic agent with a solution of a biodegradable, polymerizable macromer as claimed in any one of Claims 1 to 24: and
   b) polymerizing the macromer to incorporate the agent within the resulting polymer.

28. A method according to Claim 27, wherein the controlled release device is formed on the surface of a medical device.

29. The use of a solution of a macromer as defined in Claim 25 for the manufacture of a medicament for making a device for controlled release of a prophylactic, therapeutic or diagnostic agent.

30. A method for forming a macromer according to any one of Claims 1 to 24 comprising:

   a) reacting a trimethylene carbonate with a biocompatible compound comprising at least two hydroxyl groups to form a carbonate-comprising precursor for a sufficient time to ensure completion of the reaction and attainment of equilibrium among reacting species;
   b) adding an excess of a reagent forming a biodegradable linkage, wherein the reagent comprises a biodegradable moiety other than a carbonate: and then
   c) adding an additional reagent which forms a chemically-reactive group on the macromer.

31. Use of an aqueous solution or suspension of a polymerizable macromer as claimed in any one of Claims 1 to 24 for the manufacture of a medicament for forming a compliant polymeric material on a surface wherein the medicament is for application to the surface and the macromer is for polymerization on the surface, and wherein the normalized compliance ratio of the surface and the material is in the range of about 0.05 to about 3.

32. A biodegradable polymer obtainable from a biodegradable, polymerizable macromer as claimed in any one of Claims 1 to 24.

33. A compliant biodegradable, polymeric material for use in medicine, obtainable by polymerization of the macromer of any one of Claims 1 to 24, wherein the material has a normalized compliance ratio in a range of 0.05 to 3.

34. Use of a polymer according to Claim 32 in the manufacture of a medicament for sealing leaks in tissue.

**Patentansprüche**

1. Biologisch abbaubares, polymerisierbares Makromer mit einer Löslichkeit von mindestens 1 g/l in einer wässrigen Lösung bei einer Temperatur im Bereich zwischen 0 und 50 °C, das mindestens einen wasserlöslichen Bereich, mindestens einen biologisch abbaubaren Bereich und mindestens eine reaktive polymerisierbare Gruppe oder einen polymerisierbaren Bereich mit der Fähigkeit zur Vernetzung mit anderen Makromeren umfasst, wobei die polymerisierbaren Bereiche voneinander durch mindestens einen biologisch abbaubaren Bereich getrennt sind und wobei mindestens ein biologisch abbaubarer Bereich eine Trimethylencarbonat- oder Dioxanonverknüpfung umfasst.

2. Makromer nach Anspruch 1, wobei der wasserlösliche Bereich an einen biologisch abbaubaren Bereich gebunden ist und mindestens eine polymerisierbare Gruppe oder ein polymerisierbarer Bereich an den biologisch abbaubaren Bereich gebunden ist.

3. Makromer nach Anspruch 1, wobei der wasserlösliche Bereich einen zentralen Kern bildet, mindestens zwei biologisch abbaubare Bereiche an den Kern gebunden sind und mindestens zwei polymerisierbare Gruppen oder Bereiche an die biologisch abbaubaren Bereiche gebunden sind.

4. Makromer nach Anspruch 1, wobei der biologisch abbaubare Bereich ein zentraler Kern ist und mindestens eine polymerisierbare Gruppe oder ein polymerisierbarer Bereich an den Kern so gebunden ist, dass jede polymerisierbare Gruppe oder jeder polymerisierbare Bereich von jeder anderen polymerisierbaren Gruppe oder jedem anderen polymerisierbaren Bereich durch mindestens einen biologisch abbaubaren Bereich getrennt ist.

5. Makromer nach Anspruch 1, wobei der wasserlösliche Bereich ein Makromergerüst ist, der biologisch abbaubare Bereich ein Ast oder Pfropfteil, die an das Makromergerüst gebunden sind, ist und mindestens zwei polymerisierbare Gruppen oder Bereiche an die biologisch abbaubaren Bereiche gebunden sind.

6. Makromer nach Anspruch 4, das zwei oder mehrere polymerisierbare Gruppen oder Bereiche, die an den Kern gebunden sind, aufweist.

7. Makromer nach Anspruch 1, wobei der wasserlösliche Bereich ein Sterngerüst ist, der biologisch abbaubare Bereich ein Ast oder Pfropfteil, die an das wasserlösliche Sterngerüst gebunden sind, ist und mindestens zwei polymerisierbare Gruppen oder Bereiche an einen oder mehrere biologisch abbaubare Äste oder Pfropfteile gebunden sind, so dass jede polymerisierbare Gruppe oder jeder polymerisierbare Bereich von jeder anderen polymerisierbaren Gruppe oder jedem anderen polymerisierbaren Bereich durch mindestens einen biologisch abbaubaren Bereich getrennt ist.

8. Makromer nach Anspruch 1, wobei der biologisch abbaubare Bereich ein Sterngerüst ist, der wasserlösliche Bereich ein Ast oder Pfropfelement, die an das biologisch abbaubare Sterngerüst gebunden sind, ist und zwei oder mehrere polymerisierbare Gruppen an das biologisch abbaubare Gerüst so gebunden sind, dass jede polymerisierbare Gruppe oder jeder polymerisierbare Bereich von jeder anderen polymerisierbaren Gruppe oder jedem anderen polymerisierbaren Bereich durch mindestens einen biologisch abbaubaren Bereich getrennt ist.

9. Makromer nach Anspruch 1, wobei der wasserlösliche Bereich auch der biologisch abbaubare Bereich ist.

10. Makromer nach Anspruch 1, wobei der wasserlösliche Bereich auch der biologisch abbaubare Bereich ist und ein oder mehrere weitere biologisch abbaubare Bereiche Pfropfelemente oder Äste an dem wasserlöslichen Bereich sind.

11. Makromer nach Anspruch 1, das einen wasserlöslichen Kern, mindestens zwei biologisch abbaubare Verlängerungsteile am Kern und eine Endkappe an mindestens zwei Verlängerungsteilen umfasst,
wobei der Kern Poly(ethylenglykol) umfasst,
mindestens eines der Verlängerungsteile eine biologisch abbaubare Trimethylencarbonat- oder Dioxanonverknüpfung umfasst und
jede Endkappe eine Gruppe oder einen Bereich, die durch eine Reaktion mit freien Radikalen polymerisierbar sind, umfasst.

12. Makromer nach Anspruch 11, wobei mindestens eines der Verlängerungsteile eine biologisch abbaubare Poly(hydroxysäure) umfasst.

13. Makromer nach Anspruch 12, wobei das Poly(ethylenglykol) ein Molekulargewicht zwischen 400 und 40000 Da aufweist,
die Poly(hydroxysäure)-Oligomere ein Molekulargewicht zwischen 200 und 2000 Da aufweisen und
die polymerisierbare Gruppe oder der polymerisierbare Bereich ein Molekulargewicht zwischen 50 und 200 Da aufweist.

14. Makromer nach Anspruch 13, wobei die
Poly(ethylenglykol)-Oligomere ein Molekulargewicht von etwa 20000 Da aufweisen, die Poly(hydroxysäure)-Oligomere ein Molekulargewicht von etwa 1000 Da aufweisen und die polymerisierbaren Gruppen ein Molekulargewicht von etwa 50 Da aufweisen.

15. Makromer nach Anspruch 1, wobei die polymerisierbare Gruppe eine Kohlenstoff-Kohlenstoff-Doppelbindung enthält, die zur Vernetzung und Polymerisation von Makromeren fähig ist.

16. Makromer nach Anspruch 1, wobei die Vernetzung und Polymerisation des Makromers durch einen lichtempfindlichen Radikalkettenpolymerisationsinitiator mit einem oder ohne einen Co-Katalysator, der ferner einen Radikalkettenpolymerisationsinitiator umfasst, initiiert werden.

17. Makromer nach Anspruch 16, wobei der Initiator aus der Gruppe von Xanthinfarbstoffen, Acridinfarbstoffen, Thiazinfarbstoffen, Phenazinfarbstoffen, Campherchinonfarbstoffen und Acetophenonfarbstoffen ausgewählt ist.

18. Makromer nach Anspruch 17, wobei der Initiator aus der Gruppe von Eosin, Ethyleosin, 2,2-Dimethyl-2-phenylacetophenon und 2-Methoxy-2-phenylacetophenon ausgewählt ist.

19. Makromer nach Anspruch 1, wobei Vernetzung oder Polymerisationen in situ durch Licht mit einer Wellenlänge von 320 nm oder länger initiiert werden.

20. Makromer nach Anspruch 1, wobei mindestens ein biologisch abbaubarer Bereich aus der Gruppe von Poly(alphahydroxysäuren), Poly(lactonen), Poly(aminosäuren), Poly(anhydriden), Poly(orthoestern) und Poly(phosphoestern) ausgewählt ist.

21. Makromer nach Anspruch 20, wobei die Poly(alphahydroxysäure) aus der Gruppe von Poly(glykolsäure), Poly(D, L-milchsäure) und Poly(L-milchsäure) ausgewählt ist.

22. Makromer nach Anspruch 20, wobei das Poly(lacton) aus der Gruppe von Poly(epsilon-caprolacton), Poly(deltavalerolacton) und Poly(gamma-butyrolacton) ausgewählt ist.

23. Makromer nach Anspruch 1, wobei der wasserlösliche Bereich aus der Gruppe von Poly(ethylenglykol), Poly(ethylenoxid), Poly(vinylalkohol), Poly(vinylpyrrolidon), Poly(ethyloxazolin), Poly(ethylenoxid)-co-Poly(propylenoxid)-Blockcopolymeren, Polysacchariden, Kohlenhydraten, Proteinen und Kombinationen derselben ausgewählt ist.

24. Makromer nach Anspruch 1, das ferner ein prophylaktisches, therapeutisches oder diagnostisches Mittel umfasst.

25. Lösung eines biologisch abbaubaren, polymerisierbaren Makromers gemäß einem der Ansprüche 1 bis 24 zur Verwendung in der Medizin.

26. Verwendung einer Lösung eines Makromers gemäß der Definition in Anspruch 25 zur Herstellung eines Medikaments zur Bildung eines biologisch kompatiblen Überzugs auf einem Gewebe, wobei die Gewebeoberfläche einen Polymerisationsinitiator mit der Fähigkeit zum Initiieren einer Polymerisation über eine Radikalketten- oder kationische Polymerisation umfasst.

27. Verfahren zur Herstellung einer Vorrichtung zur gesteuerten Freisetzung eines prophylaktischen, therapeutischen oder diagnostischen Mittels, das umfasst:

a) Mischen eines prophylaktischen, therapeutischen oder diagnostischen Mittels mit einer Lösung eines biologisch abbaubaren, polymerisierbaren Makromers gemäß einem der Ansprüche 1 bis 24 und
b) Polymerisieren des Makromers zur Einarbeitung des Mittels in das gebildete Polymer.

28. Verfahren gemäß Anspruch 27, wobei die Vorrichtung mit gesteuerter Freisetzung auf der Oberfläche einer medizinischen Vorrichtung gebildet wird.

29. Verwendung einer Lösung eines Makromers gemäß der Definition in Anspruch 25 zur Herstellung eines Medikaments zur Herstellung einer Vorrichtung zur gesteuerten Freisetzung eines prophylaktischen, therapeutischen oder diagnostischen Mittels.

30. Verfahren zur Bildung eines Makromers nach einem der Ansprüche 1 bis 24, das umfasst:

a) Umsetzung eines Trimethylencarbonats mit einer biologisch kompatiblen Verbindung, die mindestens zwei Hydroxylgruppen umfasst, zur Bildung einer ein Carbonat umfassenden Vorstufe über einen ausreichenden Zeitraum, um die Vollständigkeit der Reaktion und das Erreichen eines Gleichgewichts der reagierenden Spezies sicherzustellen;

b) Zugabe eines Überschusses eines Reagens, das eine biologisch abbaubare Verknüpfung bildet, wobei das Reagens eine andere biologisch abbaubare Einheit als ein Carbonat umfasst; und dann

c) Zugabe eines zusätzlichen Reagens, das eine chemisch reaktive Gruppe an dem Makromer bildet.

31. Verwendung einer wässrigen Lösung oder Suspension eines polymerisierbaren Makromers gemäß einem der Ansprüche 1 bis 24 zur Herstellung eines Medikaments zur Bildung eines gefügigen polymeren Materials auf einer Oberfläche, wobei das Medikament zur Applikation auf die Oberfläche dient und das Makromer zur Polymerisation auf der Oberfläche dient und wobei das genormte-Compliance-Verhältnis der Oberfläche und des Materials im Bereich von etwa 0,05 bis etwa 3 liegt.

32. Biologisch abbaubares Polymer, das aus einem biologisch abbaubaren, polymerisierbaren Makromer gemäß einem der Ansprüche 1 bis 24 erhältlich ist.

33. Gefügiges, biologisch abbaubares, polymeres Material zur Verwendung in der Medizin, das durch Polymerisation des Makromers nach einem der Ansprüche 1 bis 24 erhältlich ist, wobei das Material ein genormte-Compliance-Verhältnis im Bereich von 0,05 bis 3 aufweist.

34. Verwendung eines Polymers gemäß Anspruch 32 bei der Herstellung eines Medikaments zur Versiegelung von Lecks in Gewebe.

**Revendications**

1. Macromère polymérisable biodégradable ayant une solubilité d'au moins un gramme/litre dans une solution aqueuse à une température dans la gamme entre 0 et 50 °C, comprenant au moins une région hydrosoluble, au moins une région biodégradable, et au moins un groupe polymérisable ou région polymérisable réactifs aptes à se réticuler avec d'autres macromères, dans lequel les régions polymérisables sont séparées les unes des autres par au moins une région biodégradable, et dans lequel au moins une région biodégradable comprend une liaison carbonate de triméthylène ou dioxanone.

2. Macromère selon la revendication 1, dans lequel la région hydrosoluble est attachée à une région biodégradable, et au moins un groupe ou région polymérisable est attaché à la région biodégradable.

3. Macromère selon la revendication 1, dans lequel la région hydrosoluble forme un coeur central, au moins deux régions biodégradables sont attachées au coeur, et au moins deux groupes ou régions polymérisables sont attachés aux régions biodégradables.

4. Macromère selon la revendication 1, dans lequel la région biodégradable est un coeur central, et au moins un groupe ou région polymérisable est attaché au coeur de sorte que chaque groupe ou région polymérisable est séparé de chaque autre groupe ou région polymérisable par au moins une région biodégradable.

5. Macromère selon la revendication 1, dans lequel la région hydrosoluble est un squelette de macromère, la région biodégradable est une ramification ou un greffon attaché au squelette du macromère, et au moins deux groupes ou régions polymérisables sont attachés aux régions biodégradables.

6. Macromère selon la revendication 4, ayant deux groupes ou régions polymérisables ou plus attachés au coeur.

7. Macromère selon la revendication 1, dans lequel la région hydrosoluble est un squelette en étoile, la région biodégradable est une ramification ou un greffon attaché au squelette en étoile hydrosoluble, et au moins deux groupes ou régions polymérisables sont attachés à une ou plusieurs ramifications ou greffons biodégradables, de sorte que chaque groupe ou région polymérisable est séparé de chaque autre groupe ou région polymérisable par au moins une région biodégradable.

8. Macromère selon la revendication 1, dans lequel la région biodégradable est un squelette en étoile, la région hydrosoluble est une ramification ou un greffon attaché au squelette en étoile biodégradable, et deux groupes polymérisables ou plus sont attachés au squelette biodégradable de sorte que chaque groupe ou région polymérisable est séparé de chaque autre groupe ou région polymérisable par au moins une région biodégradable.

9. Macromère selon la revendication 1, dans lequel la région hydrosoluble est aussi la région biodégradable.

10. Macromère selon la revendication 1, dans lequel la région hydrosoluble est aussi la région biodégradable, et une ou plusieurs régions biodégradables supplémentaires sont des greffons ou des ramifications sur la région hydrosoluble.

11. Macromère selon la revendication 1, comprenant un coeur hydrosoluble, au moins deux extensions biodégradables sur le coeur, et un capuchon terminal sur au moins deux extensions, dans lequel
le coeur comprend un poly(éthylèneglycol) ;
au moins l'une des extensions comprend une liaison carbonate de triméthylène ou dioxanone biodégradable ; et
chaque capuchon terminal comprend un groupe ou une région qui est polymérisable par une réaction radicalaire.

12. Macromère selon la revendication 11, dans lequel au moins l'une des extensions comprend un poly(hydroxy acide) biodégradable.

13. Macromère selon la revendication 12, dans lequel le poly(éthylène glycol) a un poids moléculaire entre 400 et 40 000 Da ;
les oligomères de poly(hydroxy acide) ont un poids moléculaire entre 200 et 2 000 Da ; et
le groupe ou la région polymérisable a un poids moléculaire entre 50 et 200 Da.

14. Macromère selon la revendication 13, dans lequel les oligomères de poly(éthylèneglycol) ont un poids moléculaire d'environ 20 000 Da ; les oligomères de poly(hydroxy acide) ont un poids moléculaire d'environ 1 000 Da ; et les groupes polymérisables ont un poids moléculaire d'environ 50 Da.

15. Macromère selon la revendication 1, dans lequel le groupe polymérisable contient une double liaison carbone-carbone capable de réticuler et de polymériser des macromères.

16. Macromère selon la revendication 1, dans lequel la réticulation et la polymérisation du macromère sont amorcées par un amorceur de polymérisation radicalaire photosensible, avec ou sans cocatalyseur, comprenant en outre un amorceur de polymérisation radicalaire.

17. Macromère selon la revendication 16, dans lequel l'amorceur est choisi dans le groupe constitué par les colorants xanthines, les colorants acridines, les colorants thiazines, les colorants phénazines, les colorants camphroquinones et les colorants acétophénones.

18. Macromère selon la revendication 17, dans lequel l'amorceur est choisi dans le groupe constitué par l'éosine, l'éthyl éosine, la 2,2-diméthyl-2-phényl acétophénone et la 2-méthoxy-2-phényl acétophénone.

19. Macromère selon la revendication 1, dans lequel la réticulation ou les polymérisations sont amorcées *in situ* par une lumière ayant une longueur d'onde de 320 nm ou plus.

20. Macromère selon la revendication 1, dans lequel au moins une région biodégradable est choisie dans le groupe constitué par les poly(alpha-hydroxy acides), les poly(lactones), les poly(acides aminés), les poly-(anhydrides), les poly(orthoesters) et les poly(phosphoesters).

21. Macromère selon la revendication 20, dans lequel le poly(alpha-hydroxy acide) est choisi dans le groupe constitué par un poly(acide glycolique), un poly(acide D,L-lactique) et un poly(acide L-lactique).

22. Macromère selon la revendication 20, dans lequel la poly(lactone) est choisie dans le groupe constitué par une poly (epsilon-caprolactone), une poly(delta-valérolactone) et une poly(gamma-butyrolactone).

23. Macromère selon la revendication 1, dans lequel la région hydrosoluble est choisie dans le groupe constitué par un poly(éthylèneglycol), un poly(oxyde d'éthylène), un poly(alcool vinylique), une poly(vinylpyrrolidone), une poly (éthyloxazoline), les copolymères séquencés poly(oxyde d'éthylène)-co-poly(oxyde de propylène), les polysaccharides, les glucides, les protéines, et les combinaisons de ceux-ci.

**24.** Macromère selon la revendication 1, comprenant en outre un agent prophylactique, thérapeutique ou diagnostique.

**25.** Solution d'un macromère polymérisable biodégradable tel que revendiqué dans l'une quelconque des revendications 1 à 24, pour une utilisation en médecine.

**26.** Utilisation d'une solution d'un macromère telle que définie dans la revendication 25, pour la fabrication d'un médicament pour former un revêtement biocompatible sur un tissu, dans laquelle la surface du tissu comprend un amorceur de polymérisation capable d'amorcer une polymérisation par polymérisation radicalaire ou cationique.

**27.** Procédé pour fabriquer un dispositif pour la libération contrôlée d'un agent prophylactique, thérapeutique ou diagnostique, comprenant :

a) le mélange d'un agent prophylactique, thérapeutique ou diagnostique avec une solution d'un macromère polymérisable biodégradable tel que revendiqué dans l'une quelconque des revendications 1 à 24 ; et
b) la polymérisation du macromère pour incorporer l'agent à l'intérieur du polymère obtenu.

**28.** Procédé selon la revendication 27, dans lequel le dispositif à libération contrôlée est formé sur la surface d'un dispositif médical.

**29.** Utilisation d'une solution d'un macromère telle que définie dans la revendication 25 pour la fabrication d'un médicament pour fabriquer un dispositif pour la libération contrôlée d'un agent prophylactique, thérapeutique ou diagnostique.

**30.** Procédé pour former un macromère selon l'une quelconque des revendications 1 à 24, comprenant :

a) la réaction d'un carbonate de triméthylène avec un composé biocompatible comprenant au moins deux groupes hydroxyles pour former un précurseur comprenant un carbonate, pendant une durée suffisante pour assurer l'achèvement de la réaction et l'obtention de l'équilibre entre les espèces qui réagissent ;
b) l'addition d'un excès d'un réactif formant une liaison biodégradable, dans laquelle le réactif comprend un fragment biodégradable autre qu'un carbonate ; puis
c) l'addition d'un réactif supplémentaire qui forme un groupe chimiquement réactif sur le macromère.

**31.** Utilisation d'une solution ou suspension aqueuse d'un macromère polymérisable tel que revendiqué dans l'une quelconque des revendications 1 à 24 pour la fabrication d'un médicament pour former un matériau polymère conforme sur une surface, dans laquelle le médicament est à appliquer sur la surface et le macromère est à polymériser sur la surface, et dans laquelle le rapport de conformité (compliance) normalisé de la surface et du matériau est dans la gamme d'environ 0,05 à environ 3.

**32.** Polymère biodégradable susceptible d'être obtenu à partir d'un macromère polymérisable biodégradable tel que revendiqué dans l'une quelconque des revendications 1 à 24.

**33.** Matériau polymère biodégradable conforme pour une utilisation en médecine, susceptible d'être obtenu par polymérisation du macromère selon l'une quelconque des revendications 1 à 24, dans lequel le matériau a un rapport de conformité (compliance) normalisé dans la gamme de 0,05 à 3.

**34.** Utilisation d'un polymère selon la revendication 32, dans la fabrication d'un médicament pour boucher des fuites dans un tissu.

FIG. 1

$$y = 57.830 LOG(x) + 22.828$$

FIG. 2a

FIG. 2b

FIG. 3

**EP 0 927 214 B2**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5410016 A, Hubbell
- US 4938763 A, Dunn
- US 5100992 A
- US 4826945 A, Cohn
- US 4741872 A
- US 5160745 A, De Luca
- US 5527864 A, Suggs
- US 4511478 A, Nowinski
- US 5573934 A, Hubbell
- WO 9629370 A, Focal
- US 3301824 A, Hostettler
- US 4243775 A, Rosensaft
- US 4429080 A, Casey
- US 471620 A, Casey
- US 4857602 A, Casey
- US 4882168 A, Casey
- EP 0390860 B1, Boyle
- US 5066772 A, Tang
- US 5366756 A, Chesterfield
- US 5403347 A, Roby
- US 5522841 A, Roby
- US 4526938 A, Churchill
- US 2807629 A